# EUROPEAN PATENT APPLICATION

(11) **EP 4 613 192 A1**
(43) Date of publication of application: **10.09.2025**
(21) Application number: 23910868.1
(22) Date of filing: 28.12.2023
(51) Int. Cl.: A61B 5/145

(54) **BLOOD GLUCOSE MEASUREMENT METHOD, ELECTRONIC DEVICE, AND SYSTEM**

(30) Priority: 30.12.2022 CN 202211732705
(71) Applicant: Huawei Technologies Co., Ltd., Shenzhen, Guangdong 518129 (CN)
(72) Inventor: ZHANG, Jie, Shenzhen, Guangdong 518129 (CN); DONG, Wenxiao, Shenzhen, Guangdong 518129 (CN); SUN, Yuning, Shenzhen, Guangdong 518129 (CN); XIE, Songlin, Shenzhen, Guangdong 518129 (CN)
(74) Representative: Maiwald GmbH
(86) International application number: PCT/CN2023/142912
(87) International publication number: WO 2024/140954

(57) **Abstract**

A blood glucose measurement method, an electronic device (100), and a system are provided. The blood glucose measurement method includes: first determining a core temperature of a human body, determining a sensor temperature based on the core temperature, a skin temperature, and a physiological parameter, and performing temperature compensation on blood glucose measurement, to obtain a blood glucose concentration with higher accuracy.

## Description

This application claims priority to Chinese Patent Application No. 202211732705.3, filed with the China National Intellectual Property Administration on December 30, 2022 and entitled "BLOOD GLUCOSE MEASUREMENT METHOD, ELECTRONIC DEVICE, AND SYSTEM", which is incorporated herein by reference in its entirety.

### TECHNICAL FIELD

This application relates to the field of electronic technologies, and in particular, to a blood glucose measurement method, an electronic device, and a system.

### BACKGROUND

A longer diabetes course indicates poorer blood glucose control and a higher risk of complications. Ultimately, diabetes complications may cause disability or even endanger life. Possible complications include cardiovascular diseases (a coronary heart disease, a stroke, arteriosclerosis, and the like), a nerve injury (neuropathy), a kidney injury (nephropathy), an eye injury (retinopathy), a foot injury (diabetic foot), and the like. Blood glucose monitoring is an important part of diabetes management. Continuous glucose monitoring (continuous glucose monitoring, CGM) is a technology in which a glucose concentration change of a subcutaneous interstitial fluid is continuously monitored by using a glucose sensor, to provide more comprehensive blood glucose information and help understand a characteristic of the blood glucose change. A CGM electronic device can be used to implement continuous monitoring for more than seven days.

The CGM electronic device can implant a biosensor (a microneedle sensor) subcutaneously to be in contact with a tissue fluid, to determine a tissue fluid glucose concentration, and then obtain a blood glucose concentration by compensating for a delay between tissue fluid glucose and blood glucose. However, due to impact of some factors, there is always a discrepancy between the blood glucose concentration obtained by the CGM electronic device through measuring and an actual blood glucose concentration of a user.

### SUMMARY

Embodiments of this application provide a blood glucose measurement method, an electronic device, and a system. Temperature compensation is required for blood glucose measurement, and temperature measurement accuracy directly affects accuracy of the blood glucose measurement. According to the blood glucose measurement method provided in embodiments of this application, a core temperature of a human body is first determined, a sensor temperature is determined based on the core temperature, a skin temperature, and a physiological parameter, and a more accurate sensor temperature is obtained to obtain a blood glucose concentration with higher accuracy.

According to a first aspect, an embodiment of this application provides a blood glucose measurement method, applied to a first electronic device. The first electronic device includes a glucose detection sensor and a temperature detection sensor, the temperature detection sensor is configured to detect a skin temperature, the temperature detection sensor is located outside a body of a user after a detection element of the glucose detection sensor of the first electronic device is implanted into a subcutaneous tissue of the user, the glucose detection sensor is configured to detect a glucose concentration, and the method includes: The first electronic device determines a core temperature based on a first parameter after the detection element of the glucose detection sensor of the first electronic device is implanted into the subcutaneous tissue of the user, where the first parameter includes the skin temperature, the first parameter further includes a second parameter, the second parameter includes time, a location, and/or a physiological parameter of the user, the second parameter is obtained by the first electronic device from a second electronic device, and the core temperature includes a temperature of an abdominal cavity or a chest cavity of the user; the first electronic device determines a sensor temperature based on the core temperature and the skin temperature, where the sensor temperature is a temperature of the subcutaneous tissue in which the detection element is located; and the first electronic device determines a blood glucose concentration of the user based on a reading of the glucose detection sensor and the sensor temperature.

In the foregoing embodiment, the core temperature is determined based on the physiological parameter and the skin temperature, and then the sensor temperature is determined based on the core temperature and the skin temperature. Because core temperatures of different users are different in different cases, the core temperature can be accurately determined based on the physiological parameter and the skin temperature. An accurate sensor temperature can be determined based on the accurate core temperature and skin temperature, so that accuracy of determining the blood glucose concentration of the user is also higher.

With reference to some embodiments of the first aspect, in some embodiments, that the first electronic device determines a blood glucose concentration of the user based on a reading of the glucose detection sensor and the sensor temperature specifically includes: The first electronic device determines a first current intensity based on the glucose detection sensor; the first electronic device determines a second current intensity based on the sensor temperature; the first electronic device calibrates the first current intensity to a third current intensity based on the second current intensity; and the first electronic device determines the blood glucose concentration of the user based on the third current intensity.

In the foregoing embodiment, a calibration process may be implemented by using a current intensity, or a calibrated tissue fluid glucose concentration may be directly obtained. Because a mapping relationship between a tissue fluid glucose concentration and the blood glucose concentration may not be a linear mapping, an amplitude discrepancy and/or a delay caused by a temperature may change after the mapping, and are/is difficult to correct. Impact of the temperature is directly calibrated in a process of determining the tissue fluid glucose concentration, so that a subsequent calculation amount can be reduced and accuracy can be improved.

With reference to some embodiments of the first aspect, in some embodiments, that the first electronic device determines a blood glucose concentration of the user based on a reading of the glucose detection sensor and the sensor temperature specifically includes: The first electronic device determines the tissue fluid glucose concentration based on the glucose detection sensor; and the first electronic device determines the blood glucose concentration of the user based on the tissue fluid glucose concentration and the sensor temperature.

In the foregoing embodiment, the tissue fluid glucose concentration affected by the temperature is obtained, and then temperature compensation can be completed in a process of calculating the blood glucose concentration.

With reference to some embodiments of the first aspect, in some embodiments, after the first electronic device determines the blood glucose concentration of the user based on the reading of the glucose detection sensor and the sensor temperature, the method further includes: When a first condition is met, the first electronic device determines a blood glucose status based on the blood glucose concentration of the user, where the blood glucose status includes hyperglycemia and/or hypoglycemia.

In the foregoing embodiment, when the first condition is met, whether the user has hyperglycemia or hypoglycemia may be determined based on the blood glucose concentration.

With reference to some embodiments of the first aspect, in some embodiments, after the first electronic device determines the blood glucose concentration of the user based on the reading of the glucose detection sensor and the sensor temperature, the method further includes: When the first condition is not met, the first electronic device determines the blood glucose status based on historical data of the blood glucose concentration.

In the foregoing embodiment, when the first condition is not met, whether the user has hyperglycemia or hypoglycemia may not be determined based on the blood glucose concentration. Instead, the blood glucose status is determined based on the historical data of the blood glucose concentration. This is because blood glucose data may be incorrect when the first condition is not met.

With reference to some embodiments of the first aspect, in some embodiments, the first condition is that a first proportion is less than or equal to a threshold, and the first proportion is a proportion of a blood glucose concentration change caused by the sensor temperature.

With reference to some embodiments of the first aspect, in some embodiments, after the first electronic device determines a first concentration based on the reading of the glucose detection sensor and the sensor temperature, the method further includes: When a first condition is met, the first electronic device determines a third concentration based on the first concentration and a first offset, and then determines a blood glucose status based on the third concentration, where the blood glucose status includes hyperglycemia and/or hypoglycemia. When the first condition is met, the first electronic device determines the blood glucose status based on the first concentration, where the first condition is that a first proportion is less than or equal to a threshold, and the first proportion is a proportion of a blood glucose concentration change caused by the sensor temperature.

In the foregoing embodiment, when the first condition is met, the blood glucose status of the user is determined based on the first concentration. When the first condition is not met, the blood glucose status of the user is determined based on an offset and the first concentration. For example, the third concentration is obtained based on the first concentration, the offset, and the first offset, and the blood glucose status is determined based on the third concentration. Neither the third concentration nor the first concentration may represent the blood glucose concentration of the user.

With reference to some embodiments of the first aspect, in some embodiments, before the first electronic device determines the core temperature based on the first parameter after the detection element of the glucose detection sensor of the first electronic device is implanted into the subcutaneous tissue of the user, the method further includes: The first electronic device determines that an ambient temperature is within a first interval, and/or the first electronic device determines that a change rate of the ambient temperature is less than a change rate threshold.

In the foregoing embodiment, when the change rate of the ambient temperature is greater than the change rate threshold, blood glucose measurement may not be performed, or it is considered that the blood glucose data obtained through measurement may be incorrect.

According to a second aspect, an embodiment of this application provides a blood glucose measurement method, applied to a system including a first electronic device, a second electronic device, and a third electronic device. The first electronic device includes a glucose detection sensor, the third electronic device includes a temperature detection sensor, and the method includes: The first electronic device determines first blood glucose data based on the glucose detection sensor after a detection element of the glucose detection sensor of the first electronic device is implanted into a subcutaneous tissue of a user; the second electronic device receives the first blood glucose data sent by the first electronic device; the second electronic device receives a skin temperature sent by the third electronic device, where the skin temperature is determined by the third electronic device based on the temperature detection sensor; the second electronic device determines a core temperature based on the skin temperature and a first parameter, where the first parameter includes time, a location, and/or a physiological parameter of the user, and the core temperature is a temperature of an abdominal cavity or a chest cavity of the user; the second electronic device determines a sensor temperature based on the skin temperature and the core temperature, where the sensor temperature is a temperature of the subcutaneous tissue in which the detection element is located; and the second electronic device determines a blood glucose concentration of the user based on the sensor temperature and the first blood glucose data.

In the foregoing embodiment, the core temperature is determined based on the physiological parameter and the skin temperature, and then the sensor temperature is determined based on the core temperature and the skin temperature. Because core temperatures of different users are different in different cases, the core temperature can be accurately determined based on the physiological parameter and the skin temperature. An accurate sensor temperature can be determined based on the accurate core temperature and skin temperature, so that accuracy of determining the blood glucose concentration of the user is also higher.

With reference to some embodiments of the second aspect, in some embodiments, after the second electronic device receives the first blood glucose data sent by the first electronic device, the method further includes: The second electronic device sends a first message to the third electronic device; and after the third electronic device receives the first message, the third electronic device determines the skin temperature by using the temperature detection sensor.

In the foregoing embodiment, the third electronic device may be triggered to measure the skin temperature after receiving a message.

With reference to some embodiments of the second aspect, in some embodiments, after the second electronic device determines the blood glucose concentration of the user based on the sensor temperature and the first blood glucose data, the method further includes: When a first condition is met, the second electronic device determines a blood glucose status based on the blood glucose concentration of the user, where the blood glucose status includes hyperglycemia and/or hypoglycemia.

In the foregoing embodiment, when the first condition is met, whether the user has hyperglycemia or hypoglycemia may be determined based on the blood glucose concentration.

With reference to some embodiments of the second aspect, in some embodiments, after the second electronic device determines the blood glucose concentration of the user based on the sensor temperature and the first blood glucose data, the method further includes: When the first condition is not met, the second electronic device determines the blood glucose status based on historical data of the blood glucose concentration of the user.

In the foregoing embodiment, when the first condition is not met, whether the user has hyperglycemia or hypoglycemia may not be determined based on the blood glucose concentration. Instead, the blood glucose status is determined based on the historical data of the blood glucose concentration. This is because blood glucose data may be incorrect when the first condition is not met.

With reference to some embodiments of the second aspect, in some embodiments, the first condition is that a first proportion is less than or equal to a threshold, and the first proportion is a proportion of a blood glucose concentration change caused by the sensor temperature.

With reference to some embodiments of the second aspect, in some embodiments, after the second electronic device determines the blood glucose concentration of the user based on the sensor temperature and the first blood glucose data, the method further includes: When the first condition is not met, the second electronic device determines a first concentration based on the blood glucose concentration of the user and a first offset, and then determines a blood glucose status based on the first concentration, where the blood glucose status includes hyperglycemia and/or hypoglycemia; or when the first condition is met, the first electronic device determines the blood glucose status based on the blood glucose concentration of the user. The first condition is that a first proportion is less than or equal to a threshold, and the first proportion is a proportion of a blood glucose concentration change caused by the sensor temperature.

In the foregoing embodiment, when the first condition is met, the blood glucose status of the user is determined based on the first concentration. When the first condition is not met, the blood glucose status of the user is determined based on an offset and the first concentration. For example, the third concentration is obtained based on the first concentration, the offset, and the first offset, and the blood glucose status is determined based on the third concentration. Neither the third concentration nor the first concentration may represent the blood glucose concentration of the user.

With reference to some embodiments of the second aspect, in some embodiments, the first proportion is a ratio of a second concentration to the blood glucose concentration of the user, and the second concentration is a difference between the first concentration and the blood glucose concentration of the user.

With reference to some embodiments of the second aspect, in some embodiments, before the second electronic device determines the blood glucose concentration of the user based on the sensor temperature and the first blood glucose data, the method further includes: The second electronic device determines that an ambient temperature is within a first interval, and/or the second electronic device determines that a change rate of the ambient temperature is less than a change rate threshold.

In the foregoing embodiment, when the change rate of the ambient temperature is greater than the change rate threshold, blood glucose measurement may not be performed, or it is considered that the blood glucose data obtained through measurement may be incorrect.

According to a third aspect, an embodiment of this application provides a blood glucose measurement method, applied to a second electronic device. The method includes: The second electronic device receives first blood glucose data sent by a first electronic device; the second electronic device receives a skin temperature sent by the first electronic device and/or a third electronic device; the second electronic device determines a core temperature based on the skin temperature and a first parameter, where the first parameter includes time, a location, and/or a physiological parameter of a user, and the core temperature is a temperature of an abdominal cavity or a chest cavity of the user; the second electronic device determines a sensor temperature based on the skin temperature and the core temperature, where the sensor temperature is a temperature of a subcutaneous tissue in which the detection element is located; and the second electronic device determines a blood glucose concentration of the user based on the sensor temperature and the first blood glucose data.

In the foregoing embodiment, the core temperature is determined based on the physiological parameter and the skin temperature, and then the sensor temperature is determined based on the core temperature and the skin temperature. Because core temperatures of different users are different in different cases, the core temperature can be accurately determined based on the physiological parameter and the skin temperature. An accurate sensor temperature can be determined based on the accurate core temperature and skin temperature, so that accuracy of determining the blood glucose concentration of the user is also higher.

With reference to some embodiments of the third aspect, in some embodiments, after the second electronic device receives the first blood glucose data sent by the first electronic device, the method further includes: The second electronic device sends a first message to the third electronic device, where the first message is used to trigger the third electronic device to determine the skin temperature.

In the foregoing embodiment, the third electronic device may be triggered to measure the skin temperature after receiving a message.

With reference to some embodiments of the third aspect, in some embodiments, after the second electronic device determines the blood glucose concentration of the user based on the sensor temperature and the first blood glucose data, the method further includes: When a first condition is met, the second electronic device determines a blood glucose status based on the blood glucose concentration of the user, where the blood glucose status includes hyperglycemia and/or hypoglycemia.

In the foregoing embodiment, when the first condition is met, whether the user has hyperglycemia or hypoglycemia may be determined based on the blood glucose concentration.

With reference to some embodiments of the third aspect, in some embodiments, after the second electronic device determines the blood glucose concentration of the user based on the sensor temperature and the first blood glucose data, the method further includes: When the first condition is not met, the second electronic device determines the blood glucose status based on historical data of the blood glucose concentration of the user, where the blood glucose status includes hyperglycemia and/or hypoglycemia.

In the foregoing embodiment, when the first condition is not met, whether the user has hyperglycemia or hypoglycemia may not be determined based on the blood glucose concentration. Instead, the blood glucose status is determined based on the historical data of the blood glucose concentration. This is because blood glucose data may be incorrect when the first condition is not met.

With reference to some embodiments of the third aspect, in some embodiments, the first condition is that a first proportion is less than or equal to a threshold, and the first proportion is a proportion of a blood glucose concentration change caused by the sensor temperature.

With reference to some embodiments of the third aspect, in some embodiments, after the second electronic device determines the blood glucose concentration of the user based on the sensor temperature and the first blood glucose data, the method further includes: When the first condition is not met, the second electronic device determines a first concentration based on the blood glucose concentration of the user and a first offset, and then determines a blood glucose status based on the first concentration, where the blood glucose status includes hyperglycemia and/or hypoglycemia; or when the first condition is met, the first electronic device determines the blood glucose status based on the blood glucose concentration of the user. The first condition is that a first proportion is less than or equal to a threshold, and the first proportion is a proportion of a blood glucose concentration change caused by the sensor temperature.

In the foregoing embodiment, when the first condition is met, the blood glucose status of the user is determined based on the first concentration. When the first condition is not met, the blood glucose status of the user is determined based on an offset and the first concentration. For example, the third concentration is obtained based on the first concentration, the offset, and the first offset, and the blood glucose status is determined based on the third concentration. Neither the third concentration nor the first concentration may represent the blood glucose concentration of the user.

With reference to some embodiments of the third aspect, in some embodiments, the first proportion is a ratio of a second concentration to the blood glucose concentration of the user, and the second concentration is a difference between the first concentration and the blood glucose concentration of the user.

With reference to some embodiments of the third aspect, in some embodiments, before the second electronic device determines the blood glucose concentration of the user based on the sensor temperature and the first blood glucose data, the method further includes: The second electronic device determines that an ambient temperature is within a first interval, and/or the second electronic device determines that a change rate of the ambient temperature is less than a change rate threshold.

In the foregoing embodiment, when the change rate of the ambient temperature is greater than the change rate threshold, blood glucose measurement may not be performed, or it is considered that the blood glucose data obtained through measurement may be incorrect.

According to a fourth aspect, an embodiment of this application provides a first electronic device. The first electronic device includes a glucose detection sensor, a temperature detection sensor, one or more processors, and a memory. The temperature detection sensor is configured to detect a skin temperature, the temperature detection sensor is located outside a body of a user after a detection element of the glucose detection sensor of the first electronic device is implanted into a subcutaneous tissue of the user, the glucose detection sensor is configured to detect a glucose concentration, the memory is coupled to the one or more processors, the memory is configured to store computer program code, the computer program code includes computer instructions, and the one or more processors invoke the computer instructions, so that the first electronic device performs the following operations: The first electronic device determines a core temperature based on a first parameter after the detection element of the glucose detection sensor of the first electronic device is implanted into the subcutaneous tissue of the user, where the first parameter includes the skin temperature, the first parameter further includes a second parameter, the second parameter includes time, a location, and/or a physiological parameter of the user, the second parameter is obtained by the first electronic device from a second electronic device, and the core temperature is a temperature of an abdominal cavity or a chest cavity of the user; the first electronic device determines a sensor temperature based on the core temperature and the skin temperature, where the sensor temperature is a temperature of the subcutaneous tissue in which the detection element is located; and the first electronic device determines a blood glucose concentration of the user based on a reading of the glucose detection sensor and the sensor temperature.

With reference to some embodiments of the fourth aspect, in some embodiments, the one or more processors are specifically configured to invoke the computer instructions, so that the first electronic device performs the following operations: The first electronic device determines a first current intensity based on the glucose detection sensor; the first electronic device determines a second current intensity based on the sensor temperature; the first electronic device calibrates the first current intensity to a third current intensity based on the second current intensity; and the first electronic device determines the blood glucose concentration of the user based on the third current intensity.

With reference to some embodiments of the fourth aspect, in some embodiments, the one or more processors are specifically configured to invoke the computer instructions, so that the first electronic device performs the following operations: The first electronic device determines a tissue fluid glucose concentration based on the glucose detection sensor; and the first electronic device determines the blood glucose concentration of the user based on the tissue fluid glucose concentration and the sensor temperature.

With reference to some embodiments of the fourth aspect, in some embodiments, the one or more processors are further configured to invoke the computer instructions, so that the first electronic device performs the following operations: When a first condition is not met, the first electronic device determines a blood glucose status based on historical data of the blood glucose concentration.

With reference to some embodiments of the fourth aspect, in some embodiments, the first condition is that a first proportion is less than or equal to a threshold, and the first proportion is a proportion of a blood glucose concentration change caused by the sensor temperature.

With reference to some embodiments of the fourth aspect, in some embodiments, the one or more processors are further configured to invoke the computer instructions, so that the first electronic device performs the following operations: When a first condition is not met, the first electronic device determines a third concentration based on a first concentration and a first offset, and then determines a blood glucose status based on the third concentration, where the blood glucose status includes hyperglycemia and/or hypoglycemia. When the first condition is met, the first electronic device determines the blood glucose status based on the first concentration, where the first condition is that a first proportion is less than or equal to a threshold, and the first proportion is a proportion of a blood glucose concentration change caused by the sensor temperature.

With reference to some embodiments of the fourth aspect, in some embodiments, the one or more processors are specifically configured to invoke the computer instructions, so that the first electronic device performs the following operations: The first electronic device determines that an ambient temperature is within a first interval, and/or the first electronic device determines that a change rate of the ambient temperature is less than a change rate threshold.

According to a fifth aspect, an embodiment of this application provides a second electronic device. The electronic device includes one or more processors and a memory. The memory is coupled to the one or more processors, the memory is configured to store computer program code, the computer program code includes computer instructions, and the one or more processors invoke the computer instructions, so that the second electronic device performs the following operations: The second electronic device receives first blood glucose data sent by a first electronic device; the second electronic device receives a skin temperature sent by the first electronic device and/or a third electronic device; the second electronic device determines a core temperature based on the skin temperature and a first parameter, where the first parameter includes time, a location, and/or a physiological parameter of a user, and the core temperature is a temperature of an abdominal cavity or a chest cavity of the user; the second electronic device determines a sensor temperature based on the skin temperature and the core temperature, where the sensor temperature is a temperature of a subcutaneous tissue in which a detection element is located; and the second electronic device determines a blood glucose concentration of the user based on the sensor temperature and the first blood glucose data.

With reference to some embodiments of the fifth aspect, in some embodiments, the one or more processors are further configured to invoke the computer instructions, so that the second electronic device performs the following operations: The second electronic device sends a first message to the third electronic device, where the first message is used to trigger the third electronic device to determine the skin temperature.

With reference to some embodiments of the fifth aspect, in some embodiments, the one or more processors are further configured to invoke the computer instructions, so that the second electronic device performs the following operations: When a first condition is met, the second electronic device determines a blood glucose status based on the blood glucose concentration of the user, where the blood glucose status includes hyperglycemia and/or hypoglycemia.

With reference to some embodiments of the fifth aspect, in some embodiments, the one or more processors are further configured to invoke the computer instructions, so that the second electronic device performs the following operations: When the first condition is not met, the second electronic device determines the blood glucose status based on historical data of the blood glucose concentration of the user, where the blood glucose status includes hyperglycemia and/or hypoglycemia.

With reference to some embodiments of the fifth aspect, in some embodiments, the one or more processors are further configured to invoke the computer instructions, so that the second electronic device performs the following operations: When the first condition is not met, the second electronic device determines a first concentration based on the blood glucose concentration of the user and a first offset, and then determines the blood glucose status based on the first concentration, where the blood glucose status includes hyperglycemia and/or hypoglycemia; or when the first condition is met, the first electronic device determines the blood glucose status based on the blood glucose concentration of the user. The first condition is that a first proportion is less than or equal to a threshold, and the first proportion is a proportion of a blood glucose concentration change caused by the sensor temperature.

With reference to some embodiments of the fifth aspect, in some embodiments, the first proportion is a ratio of a second concentration to the blood glucose concentration of the user, and the second concentration is a difference between the first concentration and the blood glucose concentration of the user.

With reference to some embodiments of the fifth aspect, in some embodiments, the one or more processors are further configured to invoke the computer instructions, so that the second electronic device performs the following operations: The second electronic device determines that an ambient temperature is within a first interval, and/or the second electronic device determines that a change rate of the ambient temperature is less than a change rate threshold.

According to a sixth aspect, an embodiment of this application provides a chip system. The chip system is applied to an electronic device, and the chip system includes one or more processors. The processor is configured to invoke computer instructions, so that the electronic device performs the method described in the first aspect, the third aspect, any possible implementation of the first aspect, or any possible implementation of the third aspect.

According to a seventh aspect, an embodiment of this application provides a computer program product including instructions. When the computer program product runs on an electronic device, the electronic device is enabled to perform the method described in the first aspect, the third aspect, any possible implementation of the first aspect, or any possible implementation of the third aspect.

According to an eighth aspect, an embodiment of this application provides a computer-readable storage medium, including instructions. When the instructions are run on an electronic device, the electronic device is enabled to perform the method described in the first aspect, the third aspect, any possible implementation of the first aspect, or any possible implementation of the third aspect.

It may be understood that the electronic device provided in the fourth aspect, the electronic device provided in the fifth aspect, the chip system provided in the sixth aspect, the computer program product provided in the seventh aspect, and the computer storage medium provided in the eighth aspect are all configured to perform the method provided in embodiments of this application. Therefore, for beneficial effects that can be achieved by the electronic device, the chip system, the computer program product, and the computer storage medium, refer to beneficial effects in the corresponding method. Details are not described herein again.

### BRIEF DESCRIPTION OF DRAWINGS

FIG. 1A to FIG. 1D are diagrams of examples of a relationship between a tissue fluid glucose concentration and a blood glucose concentration according to an embodiment of this application;
FIG. 2A is a diagram of an example of a method for determining a blood glucose concentration according to an embodiment of this application;
FIG. 2B is a diagram of another example of a method for determining a blood glucose concentration according to an embodiment of this application;
FIG. 3A and FIG. 3B are diagrams of examples of a hardware architecture of an electronic device 100 according to an embodiment of this application;
FIG. 4A and FIG. 4B are diagrams of examples of a hardware architecture of an electronic device 200 according to an embodiment of this application;
FIG. 5 is a diagram of an example of a hardware structure of an electronic device 300 according to an embodiment of this application;
FIG. 6 is a diagram of an example of an architecture of a system according to an embodiment of this application;
FIG. 7 is a diagram of an example of a blood glucose measurement method according to an embodiment of this application;
FIG. 8A is a diagram of an example of a blood glucose measurement method according to an embodiment of this application;
FIG. 8B is a diagram of an example of a biological heat transfer model according to an embodiment of this application;
FIG. 9A is a diagram of an example of determining a temperature proportion according to an embodiment of this application;
FIG. 9B to FIG. 9D are diagrams of examples of a blood glucose concentration and a blood glucose status according to an embodiment of this application;
FIG. 10 is a diagram of another example of a blood glucose measurement method according to an embodiment of this application;
FIG. 11 is a diagram of another example of a method procedure of a blood glucose measurement method according to an embodiment of this application;
FIG. 12 is a diagram of another example of a blood glucose measurement method according to an embodiment of this application;
FIG. 13 is a diagram of another example of a blood glucose measurement method according to an embodiment of this application; and
FIG. 14 is a diagram of another example of a blood glucose measurement method according to an embodiment of this application.

### DESCRIPTION OF EMBODIMENTS

Terms used in the following embodiments of this application are merely intended to describe specific embodiments, but are not intended to limit this application. Terms "one", "a", "the", "the foregoing", "this", and "the one" of singular forms used in this specification of this application are also intended to include plural forms, unless otherwise specified in the context clearly. It should also be understood that, the term "and/or" used in this application indicates and includes any or all possible combinations of one or more of the listed items.

Terms "first" and "second" mentioned below are merely intended for description, and shall not be understood as an indication or implication of relative importance or implicit indication of a quantity of indicated technical features. Therefore, a feature limited by "first" and "second" may explicitly or implicitly include one or more features. In the descriptions of embodiments of this application, unless otherwise specified, "a plurality of" means two or more.

A term "user interface (user interface, UI)" in the following embodiments of this application is a medium interface for interaction and information exchange between an application or an operating system and a user, and implements conversion between an internal form of information and a form that can be accepted by the user. The user interface is source code written in a specific computer language like Java or an extensible markup language (extensible markup language, XML). Interface source code is parsed and rendered on an electronic device, and is finally presented as content that can be identified by the user. A frequently-used representation form of the user interface is a graphical user interface (graphical user interface, GUI), and is a user interface that is displayed in a graphical manner and that is related to a computer operation. The user interface may be a visual interface element like a text, an icon, a button, a menu, a tab, a text box, a dialog box, a status bar, a navigation bar, or a Widget that is displayed on a display of the electronic device.

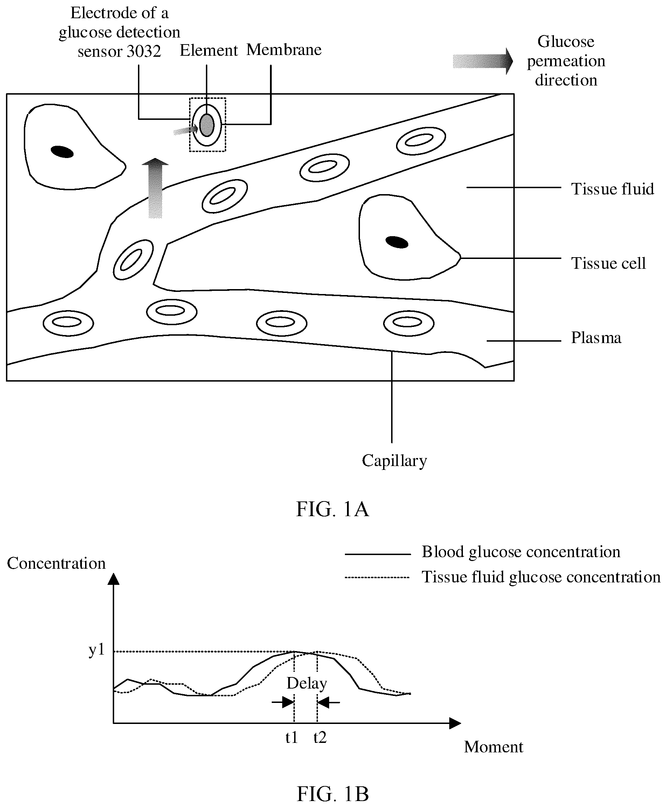

A CGM electronic device measures a tissue fluid glucose concentration (tissue fluid glucose), and then obtains, through calculation, a plasma (blood) glucose concentration (blood glucose). A relationship between the tissue fluid glucose concentration and the blood glucose concentration is shown in FIG. 1A to FIG. 1D below.

FIG. 1A to FIG. 1D are diagrams of examples of a relationship between a tissue fluid glucose concentration and a blood glucose concentration according to an embodiment of this application.

A human body transports a substance in plasma to a tissue and an organ of the body through a capillary. For example, as shown in FIG. 1A, the substance like glucose in the plasma permeates into a tissue fluid, and then can be obtained by a tissue cell. In other words, the glucose permeates from plasma in the capillary to the tissue fluid.

Because glucose permeation takes time, the tissue fluid glucose concentration is different from the blood glucose concentration at a same moment, but there is a delay. A permeation rate of the glucose is affected by plasma osmotic pressure, tissue fluid osmotic pressure, and a difference between the tissue fluid glucose concentration and the blood glucose concentration.

After a user implants a CGM electronic device into a subcutaneous tissue, the CGM electronic device measures the tissue fluid glucose concentration by using an electrode of a glucose detection sensor 3032, to determine the blood glucose concentration of the user. For the glucose detection sensor 3032, refer to a text description in the following. Details are not described herein.

The electrode of the glucose detection sensor 3032 is a component configured to measure a glucose concentration, and the tissue fluid includes various substances. Therefore, optionally, in some implementations of this application, an electrode of a glucose sensor includes an element and a membrane. The membrane may be made of various materials. This is not limited herein. The membrane may be used as a semipermeable membrane, and allows glucose to permeate freely, so that a blood glucose electrode can detect the tissue fluid glucose concentration more accurately and more quickly by using the element.

As shown in FIG. 1B, a change trend of the blood glucose concentration is similar to that of the tissue fluid glucose concentration. However, because a change of the tissue fluid glucose concentration lags behind that of the blood glucose concentration, the tissue fluid glucose concentration cannot reflect the blood glucose concentration at a same moment.

For example, in FIG. 1B, the blood glucose concentration reaches a maximum value y1 at a moment t1, and the tissue fluid glucose concentration reaches a maximum value y2 at a moment t2. In this case, in FIG. 1B, a change of the tissue fluid glucose concentration is delayed for duration of t2-t1.

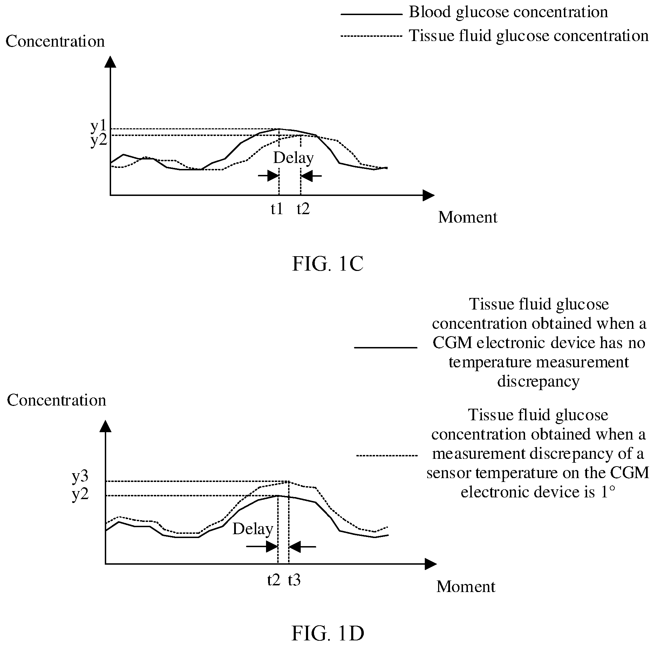

Further, if a dynamic change in the blood glucose concentration is considered and that permeation speeds of the glucose in the tissue fluid and the blood glucose are affected by osmotic pressure and a concentration difference is considered, the blood glucose concentration and the tissue fluid glucose concentration may be shown in FIG. 1C. Larger osmotic pressure indicates a faster permeation speed, and a larger concentration difference indicates a faster permeation speed. The glucose permeates into the tissue fluid, and the glucose is absorbed by the tissue cell. Compared with the blood glucose concentration, the tissue fluid glucose concentration not only has a delay, but also has a numerical difference.

The blood glucose concentration reaches a maximum value y1 at a moment t1, and the tissue fluid glucose concentration reaches a maximum value y2 at a moment t2, where y1 is less than y2. In this case, the tissue fluid glucose concentration cannot directly reflect the blood glucose concentration.

With reference to the content shown in FIG. 1B and FIG. 1C, in embodiments of this application, a mapping relationship between the blood glucose concentration and the tissue fluid glucose concentration may be established. For example, a mapping relationship between the blood glucose concentration and the tissue fluid glucose concentration is established by using a blood glucose model (a compensation model). This is not limited herein. Therefore, after determining the tissue fluid glucose concentration, the CGM electronic device or another electronic device may determine the blood glucose concentration of the user based on the tissue fluid glucose concentration.

In addition to the content shown in FIG. 1B and FIG. 1C, a temperature affects the permeation speed of the glucose, a working status of a component configured to detect the tissue fluid glucose concentration, and/or the like. As a result, the CGM electronic device cannot obtain an accurate tissue fluid glucose concentration of the user, and the CGM electronic device or the another electronic device estimates a blood glucose concentration of the user with a large discrepancy. That a temperature affects a working state of a component configured to detect the tissue fluid glucose concentration may include: When the CGM electronic device detects the tissue fluid glucose concentration by using an electrochemical method or in another manner, the temperature affects occurrence efficiency of a chemical reaction and further affects determining of the tissue fluid glucose concentration.

For example, in FIG. 1A, glucose oxidase is attached to the element, and the element may determine the tissue fluid glucose concentration by detecting a magnitude of a current generated through movement of electrons generated by an oxidation-reduction reaction.

FIG 1D is a diagram of an example of impact of the temperature on the tissue fluid glucose concentration according to an embodiment of this application.

As shown in FIG. 1D, if the CGM electronic device has no temperature measurement discrepancy, a value of the tissue fluid glucose concentration at T2 is y2; or if the CGM electronic device has a temperature measurement discrepancy, a value of the tissue fluid glucose concentration at T3 is y3.

A temperature of the CGM electronic device includes a temperature of a part of a sensor that is implanted into a subcutaneous tissue and that is used to detect the glucose. In the following, the temperature of the part of the sensor that is implanted into the subcutaneous tissue and that is used to detect the glucose is referred to as a sensor temperature.

Optionally, in some implementations of this application, the sensor temperature may be equal to a temperature of a tissue fluid into which the CGM electronic device is implanted.

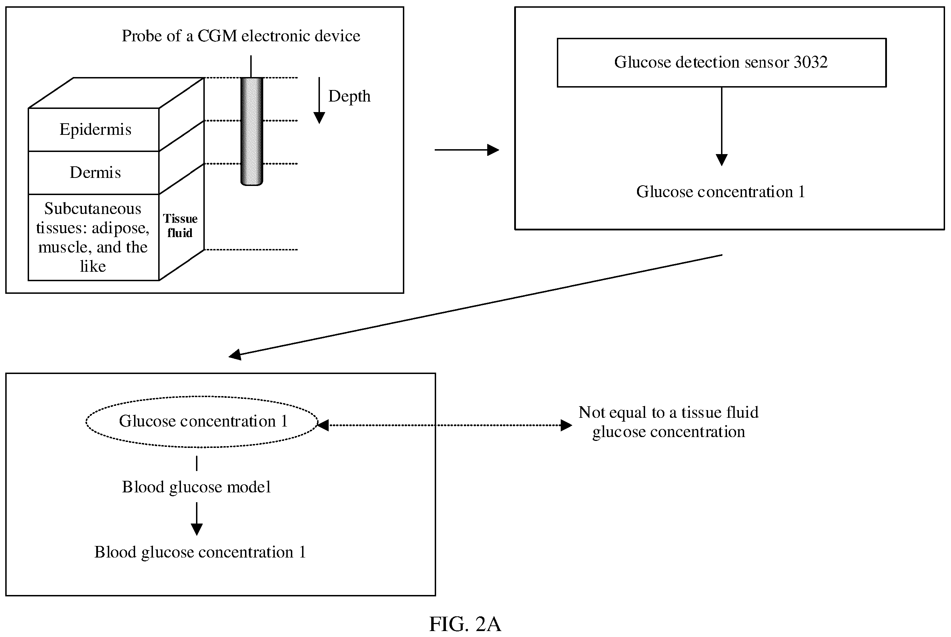

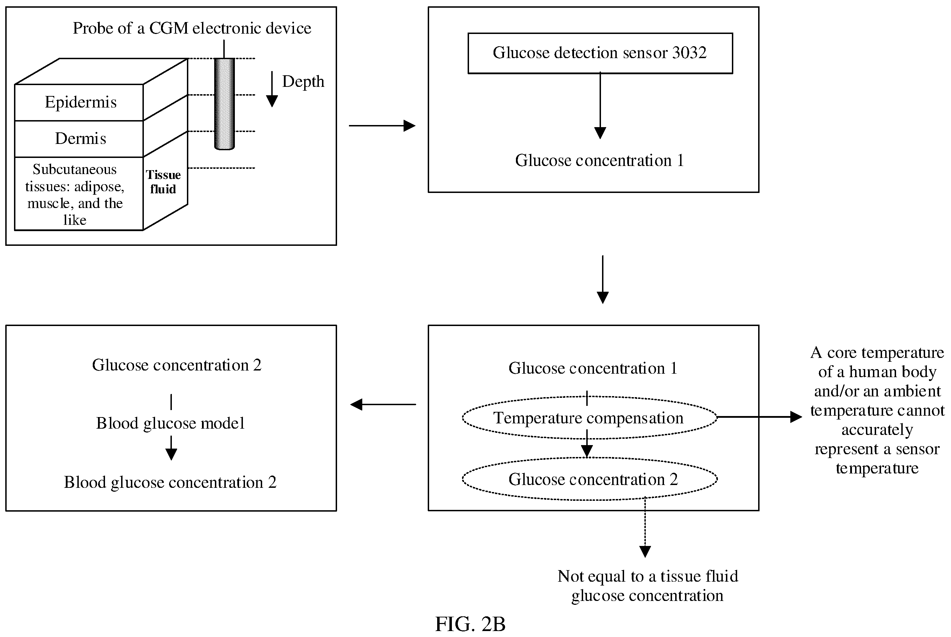

It is clear that, with reference to the content shown in FIG. 1D, the sensor temperature affects the tissue fluid glucose concentration determined by the CGM electronic device, and further affects accuracy of determining the blood glucose concentration by the CGM electronic device or the another electronic device, as shown in FIG. 2A and FIG. 2B below.

FIG. 2A is a diagram of an example of a method for determining a blood glucose concentration according to an embodiment of this application.

As shown in FIG. 2A, a CGM electronic device implants, by using a probe, a blood glucose electrode into a tissue fluid of a subcutaneous tissue, for example, an adipose layer of the subcutaneous tissue. A glucose concentration 1 determined by the CGM electronic device by using a glucose detection sensor 3032 is an estimation of a tissue fluid glucose concentration.

Due to impact of a temperature, the glucose concentration 1 is not equal to the tissue fluid glucose concentration. In this case, there is a discrepancy between a blood glucose concentration of a user and a blood glucose concentration 1 determined by the CGM electronic device or another electronic device based on the glucose concentration 1 and by using a blood glucose model.

FIG. 2B is a diagram of another example of a method for determining a blood glucose concentration according to an embodiment of this application.

A difference between content shown in FIG. 2B and content shown in FIG. 2A lies in that, after determining the glucose concentration 1, the CGM electronic device may compensate for the glucose concentration 1 as a glucose concentration 2 based on a core temperature of a human body or an ambient temperature. Then, the CGM electronic device or the another electronic device determines a blood glucose concentration 2 based on the glucose concentration 2 and by using the blood glucose model.

It is clear that, none of the core temperature of the human body, the ambient temperature, or a skin temperature can represent a sensor temperature, in other words, the core temperature of the human body, the ambient temperature, or the skin temperature does not directly affect a working state of a component configured to detect the tissue fluid glucose concentration, and the glucose concentration 2 is not equal to the tissue fluid glucose concentration. Therefore, there is a discrepancy between the blood glucose concentration 2 and the blood glucose concentration of the user.

The core temperature of the human body is affected by human metabolism, and generally refers to temperatures/a temperature of an abdominal cavity, a chest cavity, and/or a cranial cavity. In different cases, a relationship between the skin temperature and the core temperature of the human body is different. For example, when a disease like hyperthermia occurs in the human body, the core temperature of the human body may increase and the skin temperature may decrease. The core temperature of the human body is usually stable, and the skin temperature fluctuates greatly due to factors such as personal dressing and the ambient temperature.

The skin temperature may also be referred to as a shell temperature.

In view of the foregoing problems, to obtain a blood glucose concentration with more accurate time and values, embodiments of this application provide a blood glucose measurement method, an electronic device, and a system. The following first describes a hardware structure of an electronic device according to this application.

To distinguish between different electronic devices, in embodiments of this application, a CGM electronic device may be referred to as an electronic device 100, an electronic device configured to obtain a core temperature of a human body and/or an ambient temperature and/or a skin temperature is referred to as an electronic device 200, and an electronic device configured to interact with a user is an electronic device 300.

The electronic device 200 may be a watch, a band, a thermometer, or the like. This is not limited herein.

The electronic device 300 may be an electronic device having a display and/or audio playing capability. For example, the electronic device 300 is a mobile phone, a tablet computer, a desktop computer, a laptop computer, a handheld computer, a notebook computer, an ultra-mobile personal computer (ultra-mobile personal computer, UMPC), a netbook, a cellular phone, a personal digital assistant (personal digital assistant, PDA), an augmented reality (augmented reality, AR) device, a virtual reality (virtual reality, VR) device, an artificial intelligence (artificial intelligence, AI) device, a wearable device, a vehicle-mounted device, a smart home device, a smart city device, and/or the like. This is not limited herein.

Optionally, in some implementations of this application, the electronic device 200 may have a display and/or audio playing capability.

Optionally, in some implementations of this application, the electronic device 300 may further obtain a core temperature of a human body and/or an ambient temperature and/or a skin temperature.

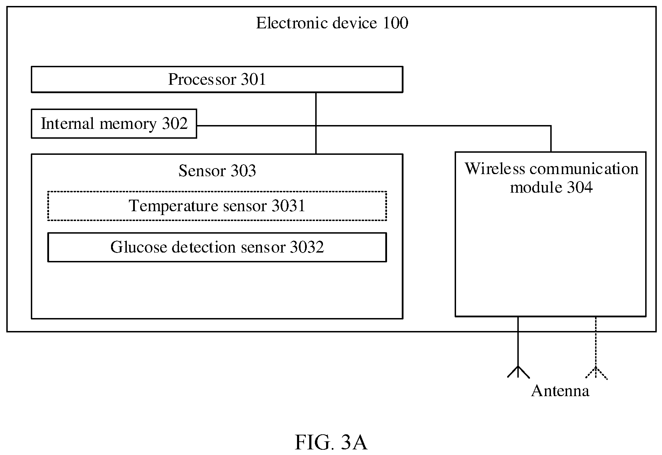

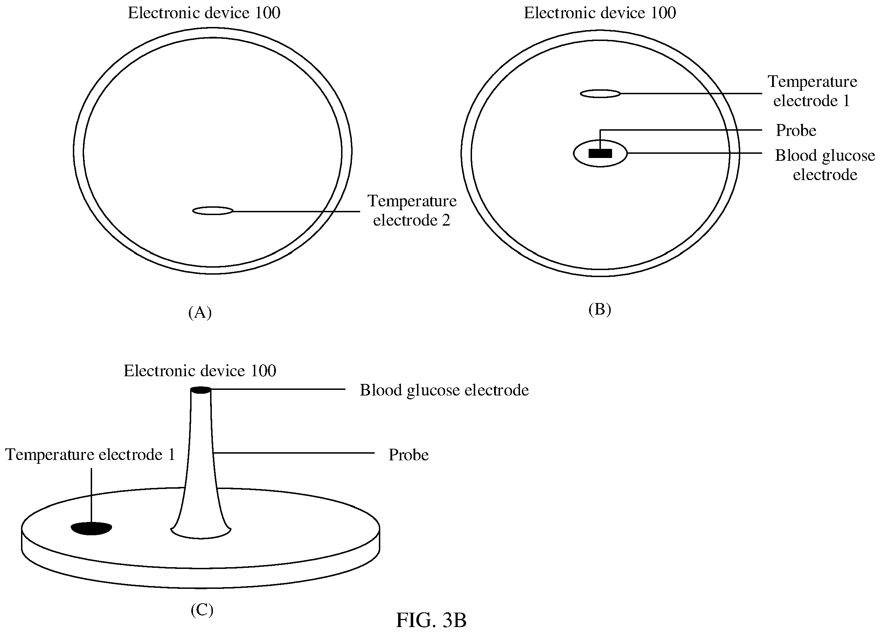

FIG. 3A and FIG. 3B are diagrams of examples of a hardware architecture of the electronic device 100 according to an embodiment of this application.

As shown in FIG. 3A, the electronic device 100 includes a processor 301, an internal memory 302, a sensor 303, a wireless communication module 304, and the like.

It may be understood that the structure illustrated in embodiments of the present invention does not constitute a specific limitation on the electronic device. In some other embodiments of this application, the electronic device may include more or fewer components than those shown in the figure, or some components may be combined, or some components may be split, or different component arrangements may be used. The components shown in the figure may be implemented by using hardware, software, or a combination of software and hardware.

The processor 301 may include one or more processing units. For example, the processor 301 may be a modem processor, a digital signal processor (digital signal processor, DSP), a controller, a baseband processor, a neural-network processing unit (neural-network processing unit, NPU), and/or the like. Different processing units may be independent components, or may be integrated into one or more processors. The processor 301 may also be referred to as a microcontroller unit (Microcontroller Unit, MCU).

The controller may generate an operation control signal based on an instruction operation code and a time sequence signal, to complete control of instruction reading and instruction execution.

A memory may be further disposed in the processor 301, and is configured to store instructions and data. In some embodiments, the memory in the processor 301 is a cache. The memory may store instructions or data just used or cyclically used by the processor 301. If the processor 301 needs to use the instructions or the data again, the processor 301 may directly invoke the instructions or the data from the memory. This avoids repeated access, reduces waiting time of the processor 301, and improves system efficiency.

In some embodiments, the processor 301 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The I2C interface is a bidirectional synchronous serial bus, and includes a serial data line (serial data line, SDA) and a serial clock line (serial clock line, SCL). The PCM interface may also be used for audio communication, and sample, quantize, and encode an analog signal. The UART interface is a universal serial data bus, and is used for asynchronous communication. The bus may be a two-way communication bus. The bus converts to-be-transmitted data between serial communication and parallel communication. The GPIO interface may be configured by using software. The GPIO interface may be configured as a control signal or a data signal. The USB interface is an interface that conforms to a USB standard specification, and may be specifically a mini USB interface, a micro USB interface, a USB type-C interface, or the like.

The wireless communication module 304 may provide a wireless communication solution that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, or the like and that is applied to the electronic device. The wireless communication module 304 may be one or more components integrating at least one communication processing module. The wireless communication module 304 receives an electromagnetic wave through an antenna, performs frequency modulation and filtering processing on an electromagnetic wave signal, and sends a processed signal to the processor 301. The wireless communication module 304 may further receive a to-be-sent signal from the processor 301, perform frequency modulation and amplification on the signal, and convert the signal into an electromagnetic wave through the antenna for radiation.

The internal memory 302 may include one or more random access memories (random access memories, RAMs) and one or more nonvolatile memories (nonvolatile memories, NVMs).

The random access memory may include a static random access memory (static random access memory, SRAM), a dynamic random access memory (dynamic random access memory, DRAM), a synchronous dynamic random access memory (synchronous dynamic random access memory, SDRAM), a double data rate synchronous dynamic random access memory (double data rate synchronous dynamic random access memory, DDR SDRAM, for example, a 5th generation DDR SDRAM, usually referred to as a DDR5 SDRAM), and the like.

The nonvolatile memory may include a magnetic disk storage device and a flash memory (flash memory).

According to an operating principle, the flash memory may be classified into NOR FLASH, NAND FLASH, 3D NAND FLASH, and the like; according to potential orders of storage cells, the flash memory may be classified into a single-level storage cell (single-level cell, SLC), a multi-level storage cell (multi-level cell, MLC), a triple-level storage cell (triple-level cell, TLC), a quad-level storage cell (quad-level cell, QLC), and the like; and according to storage specifications, the flash memory may be classified into a universal flash storage (English: universal flash storage, UFS), an embedded multi media card (embedded multi media Card, eMMC), and the like.

The random access memory may be directly read and written by the processor 301, may be configured to store an executable program (for example, a machine instruction) of an operating system or another running program, and may be further configured to store data of a user, data of an application, and the like.

The nonvolatile memory may also store an executable program, data of a user, data of an application, and the like, which may be loaded into the random access memory in advance for directly reading and writing by the processor 301.

A temperature sensor 3031 is configured to detect a temperature. In some embodiments, the electronic device 100 determines the skin temperature of the user and/or the ambient temperature by using the temperature detected by the temperature sensor 3031.

A glucose detection sensor 3032 is configured to detect a glucose concentration. In some embodiments, the glucose detection sensor 3032 determines the glucose concentration by detecting consumption of oxygen under a catalytic action of glucose oxidase or *H₂O₂* generated by a glucose oxidation reaction in a tissue fluid. In some embodiments, the glucose detection sensor 3032 connects the glucose oxidase to an electrode surface by using an electronic medium like a nanomaterial, metal osmium, ferrocene, or benzene quinone, then implements electron transfer through a series of oxidation-reduction reactions, and further determines the glucose concentration.

As shown in FIG. 3B, the electronic device 100 may include a plurality of electrodes. For example, three electrodes in FIG. 3B are respectively a temperature electrode 1, a temperature electrode 2, and a blood glucose electrode. The blood glucose electrode is located at a bottom of a probe, so that the blood glucose electrode can be implanted into a subcutaneous tissue fluid.

As shown in (A) in FIG. 3B, the temperature electrode 2 is located on a side that is of the electronic device and that is away from a skin. The temperature electrode 2 may be configured to measure the ambient temperature.

As shown in (B) and (C) in FIG. 3B, the temperature electrode 1 and the blood glucose electrode are located on a side close to the skin. The temperature electrode 1 is configured to measure the skin temperature. The blood glucose electrode is configured to measure a tissue fluid glucose concentration. For example, after the user implants the electronic device 100, the temperature electrode 1 is in contact with the skin of the user, to measure the skin temperature.

Optionally, in some implementations of this application, the electronic device 100 may not include the temperature electrode 1 and the temperature electrode 2, but include only a temperature electrode. In this case, a CGM electronic device obtains the ambient temperature and/or the core temperature of the human body by using another electronic device like the electronic device 200 or the electronic device 300. Alternatively, in this case, after determining the glucose concentration, a CGM electronic device sends the glucose concentration to another electronic device, and then the another electronic device performs sensor temperature-based compensation on the glucose temperature to obtain the tissue fluid glucose concentration.

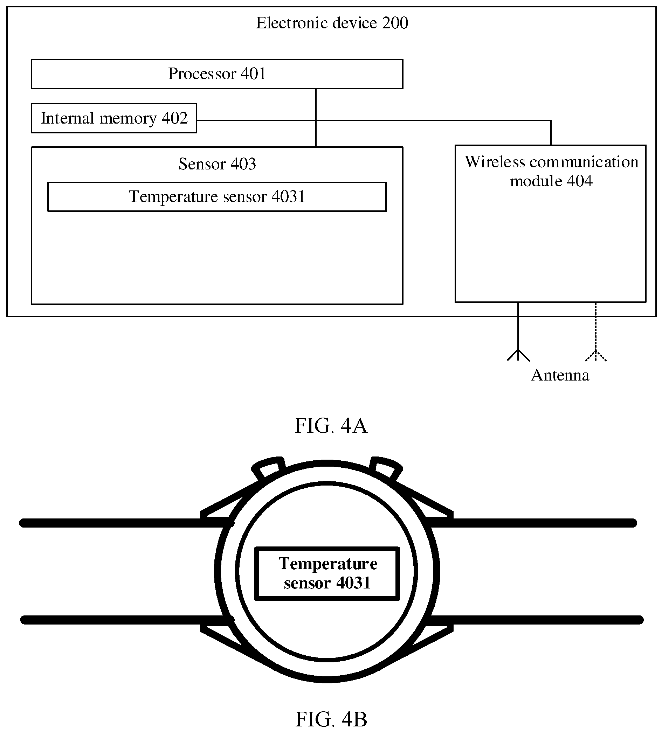

FIG. 4A and FIG. 4B are diagrams of examples of a hardware architecture of the electronic device 200 according to an embodiment of this application.

As shown in FIG. 4A, the electronic device 200 includes a processor 401, an internal memory 402, a sensor 403, a wireless communication module 404, and the like. For the processor 401, refer to the foregoing text description of the processor 301. Details are not described herein again. For the internal memory 402, refer to the foregoing text description of the internal memory 302. Details are not described herein again. For the wireless communication module 404, refer to the foregoing text description of the wireless communication module 304. Details are not described herein again.

The sensor 403 includes a temperature sensor 4031 and the like. For the temperature sensor 4031, refer to the foregoing content of the temperature sensor 3031. Details are not described herein again.

As shown in FIG. 4B, when the electronic device 200 is a watch, the temperature sensor 4031 is located on the back of a watch face of the electronic device 200. The temperature sensor 4031 may measure a skin temperature.

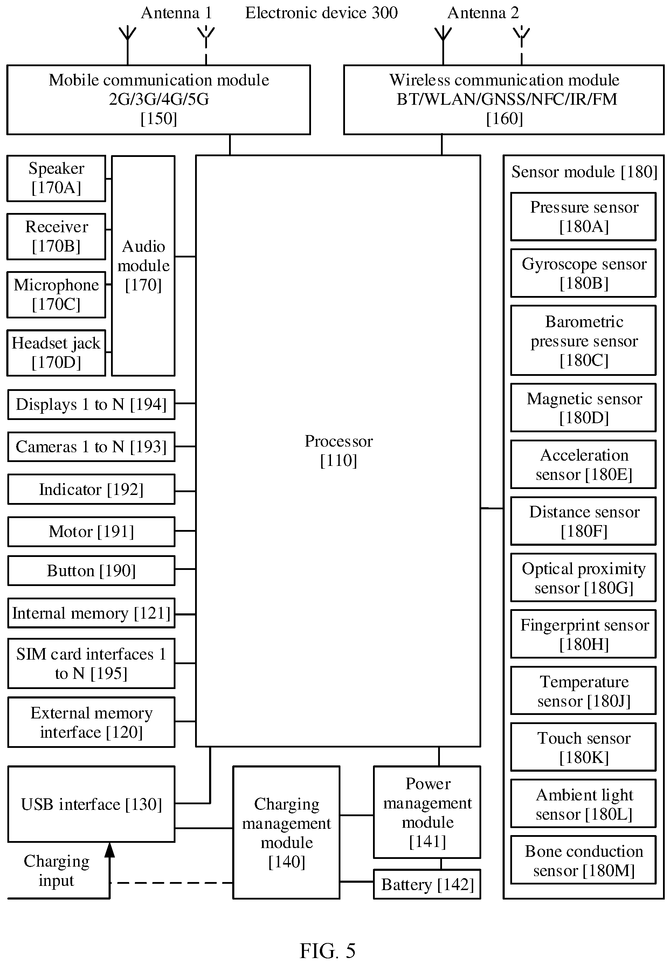

FIG. 5 is a diagram of an example of a hardware structure of the electronic device 300 according to an embodiment of this application.

The electronic device 300 may include a processor 110, an external memory interface 120, an internal memory 121, a universal serial bus (universal serial bus, USB) interface 130, a charging management module 140, a power management module 141, a battery 142, an antenna 1, an antenna 2, a mobile communication module 150, a wireless communication module 160, an audio module 170, a speaker 170A, a receiver 170B, a microphone 170C, a headset jack 170D, a sensor module 180, a button 190, a motor 191, an indicator 192, a camera 193, a display 194, a subscriber identification module (subscriber identification module, SIM) card interface 195, and the like. The sensor module 180 may include a pressure sensor 180A, a gyroscope sensor 180B, a barometric pressure sensor 180C, a magnetic sensor 180D, an acceleration sensor 180E, a distance sensor 180F, an optical proximity sensor 180G, a fingerprint sensor 180H, a temperature sensor 180J, a touch sensor 180K, an ambient light sensor 180L, a bone conduction sensor 180M, and the like.

It may be understood that the structure shown in embodiments of the present invention does not constitute a specific limitation on the electronic device 300. In some other embodiments of this application, the electronic device 300 may include more or fewer components than those shown in the figure, or some components may be combined, or some components may be split, or different component arrangements may be used. The components shown in the figure may be implemented by using hardware, software, or a combination of software and hardware.

The processor 110 may include one or more processing units. For example, the processor 110 may include an application processor (application processor, AP), a modem processor, a graphics processing unit (graphics processing unit, GPU), an image signal processor (image signal processor, ISP), a controller, a video codec, a digital signal processor (digital signal processor, DSP), a baseband processor, a neural-network processing unit (neural-network processing unit, NPU), and/or the like. Different processing units may be independent components, or may be integrated into one or more processors.

The controller may generate an operation control signal based on an instruction operation code and a time sequence signal, to complete control of instruction reading and instruction execution.

A memory may be further disposed in the processor 110, and is configured to store instructions and data. In some embodiments, the memory in the processor 110 is a cache. The memory may store instructions or data just used or cyclically used by the processor 110. If the processor 110 needs to use the instructions or the data again, the processor 110 may directly invoke the instructions or the data from the memory. This avoids repeated access, reduces waiting time of the processor 110, and improves system efficiency.

In some embodiments, the processor 110 may include one or more interfaces. The interface may include an inter-integrated circuit (inter-integrated circuit, I2C) interface, an inter-integrated circuit sound (inter-integrated circuit sound, I2S) interface, a pulse code modulation (pulse code modulation, PCM) interface, a universal asynchronous receiver/transmitter (universal asynchronous receiver/transmitter, UART) interface, a mobile industry processor interface (mobile industry processor interface, MIPI), a general-purpose input/output (general-purpose input/output, GPIO) interface, a subscriber identity module (subscriber identity module, SIM) interface, a universal serial bus (universal serial bus, USB) interface, and/or the like.

The charging management module 140 is configured to receive a charging input from a charger. The power management module 141 is configured to connect to the battery 142, the charging management module 140, and the processor 110.

A wireless communication function of the electronic device may be implemented by using the antenna 1, the antenna 2, the mobile communication module 150, the wireless communication module 160, the modem processor, the baseband processor, and the like.

The antenna 1 and the antenna 2 are configured to transmit and receive an electromagnetic wave signal. Each antenna of the electronic device may be configured to cover one or more communication frequency bands. Different antennas may be further multiplexed, to improve antenna utilization. For example, the antenna 1 may be multiplexed as a diversity antenna of a wireless local area network. In some other embodiments, the antenna may be used in combination with a tuning switch.

The mobile communication module 150 may provide a solution that is applied to the electronic device and that includes wireless communication such as 2G/3G/4G/5G. The mobile communication module 150 may include at least one filter, a switch, a power amplifier, a low noise amplifier (low noise amplifier, LNA), and the like. The mobile communication module 150 may receive an electromagnetic wave through the antenna 1, perform processing such as filtering or amplification on the received electromagnetic wave, and transmit the electromagnetic wave to the modem processor for demodulation. The mobile communication module 150 may further amplify a signal modulated by the modem processor, and convert the signal into an electromagnetic wave for radiation through the antenna 1. In some embodiments, at least some functional modules of the mobile communication module 150 may be disposed in the processor 110. In some embodiments, at least some functional modules of the mobile communication module 150 may be disposed in a same component as at least some modules of the processor 110.

The modem processor may include a modulator and a demodulator. The modulator is configured to modulate a to-be-sent low-frequency baseband signal into a medium-high frequency signal. The demodulator is configured to demodulate a received electromagnetic wave signal into a low-frequency baseband signal. Then, the demodulator transmits the low-frequency baseband signal obtained through demodulation to the baseband processor for processing. The low-frequency baseband signal is processed by the baseband processor and then transmitted to the application processor. The application processor outputs a sound signal through an audio device (which is not limited to the speaker 170A, the receiver 170B, or the like), or displays an image or a video through the display 194. In some embodiments, the modem processor may be an independent component. In some other embodiments, the modem processor may be independent of the processor 110, and is disposed in a same component as the mobile communication module 150 or another functional module.

The wireless communication module 160 may provide a wireless communication solution that includes a wireless local area network (wireless local area network, WLAN) (for example, a wireless fidelity (wireless fidelity, Wi-Fi) network), Bluetooth (Bluetooth, BT), a global navigation satellite system (global navigation satellite system, GNSS), frequency modulation (frequency modulation, FM), near field communication (near field communication, NFC), an infrared (infrared, IR) technology, or the like and that is applied to the electronic device. The wireless communication module 160 may be one or more components integrating at least one communication processing module. The wireless communication module 160 receives an electromagnetic wave through the antenna 2, performs frequency modulation and filtering processing on an electromagnetic wave signal, and sends a processed signal to the processor 110. The wireless communication module 160 may further receive a to-be-sent signal from the processor 110, perform frequency modulation and amplification on the signal, and convert the signal into an electromagnetic wave for radiation through the antenna 2.

The electronic device implements a display function by using the GPU, the display 194, the application processor, and the like. The GPU is a microprocessor for image processing, and is connected to the display 194 and the application processor.

The display 194 is configured to display an image, a video, and the like. The camera 193 is configured to capture a static image or a video.

The internal memory 121 may include one or more random access memories (random access memories, RAMs) and one or more nonvolatile memories (nonvolatile memories, NVMs). The external memory interface 120 may be configured to connect to an external nonvolatile memory, to extend a storage capability of the electronic device.

The electronic device may implement an audio function by using the audio module 170, the speaker 170A, the receiver 170B, the microphone 170C, the headset jack 170D, the application processor, and the like.

The audio module 170 is configured to convert digital audio information into an analog audio signal for output, and is also configured to convert an analog audio input into a digital audio signal. The speaker 170A, also referred to as a "loudspeaker", is configured to convert an audio electrical signal into a sound signal. The receiver 170B, also referred to as an "earpiece", is configured to convert an electrical audio signal into a sound signal. The microphone 170C, also referred to as a "mike" or a "mic", is configured to convert a sound signal into an electrical signal. The headset jack 170D is configured to connect to a wired headset. The pressure sensor 180A is configured to sense a pressure signal, and can convert the pressure signal into an electrical signal. The gyroscope sensor 180B may be configured to determine a motion gesture of the electronic device. The barometric pressure sensor 180C is configured to measure barometric pressure. The magnetic sensor 180D includes a Hall sensor. The acceleration sensor 180E may detect accelerations in various directions (usually on three axes) of the electronic device. The distance sensor 180F is configured to measure a distance. The electronic device may measure a distance in an infrared manner or a laser manner. The optical proximity sensor 180G may include, for example, a light emitting diode (LED) and an optical detector, for example, a photodiode. The ambient light sensor 180L is configured to sense ambient light brightness. The fingerprint sensor 180H is configured to collect a fingerprint. The temperature sensor 180J is configured to detect a temperature. The touch sensor 180K is also referred to as a "touch component". The bone conduction sensor 180M may obtain a vibration signal. The button 190 includes a power button, a volume button, and the like. The motor 191 may generate a vibration prompt. The indicator 192 may be an indicator light, and may be configured to indicate a charging status and a power change, or may be configured to indicate a message, a missed call, a notification, and the like.

Optionally, in some implementations of this application, the sensor module 180 further includes an infrared sensor. The infrared sensor is configured to detect a skin temperature, an ambient temperature, and the like. Optionally, when the electronic device 300 is a mobile phone, the infrared sensor may be located in a rear-facing camera module and/or a front-facing camera module of the mobile phone.

The SIM card interface 195 is configured to connect to a SIM card. The SIM card may be inserted into the SIM card interface 195 or removed from the SIM card interface 195, to implement contact with or separation from the electronic device.

Optionally, in some embodiments of this application, the temperature sensor 180J is configured to detect the skin temperature, the ambient temperature, and the like.

After hardware structures of the electronic device 100, the electronic device 200, and the electronic device 300 provided in embodiments of this application are described, a system provided in embodiments of this application is described.

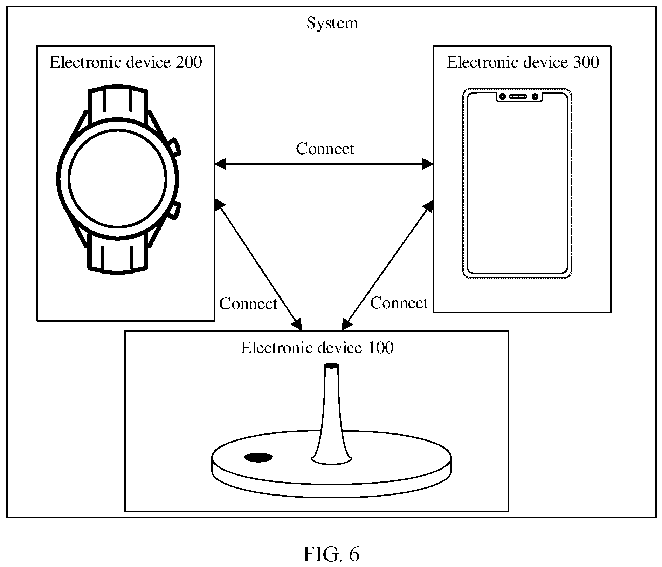

FIG. 6 is a diagram of an example of an architecture of a system according to an embodiment of this application.

As shown in FIG. 6, the system provided in embodiments of this application includes the electronic device 100, the electronic device 200, and the electronic device 300. A connection is established between every two of the electronic device 100, the electronic device 200, and the electronic device 300. Alternatively, a connection is established between the electronic device 200 and the electronic device 300, and a connection is established between the electronic device 300 and the electronic device 100.

The foregoing connection may be established in a manner like Wi-Fi, Bluetooth, near field communication (near field communication, NFC), ZigBee, Apple wireless direct link (Apple Wireless Direct Link, AWDL), or HiLink. This is not limited herein.

After the system provided in embodiments of this application is described, a blood glucose measurement method provided in this application is described.

According to the blood glucose measurement method provided in this application, a core temperature of a human body of a user is first estimated based on parameters such as an ambient temperature, a skin temperature, and/or a physiological parameter of the user, after the core temperature of the human body of the user is obtained, a sensor temperature is determined based on the core temperature of the human body and the skin temperature of the user, and further, an accurate tissue fluid glucose concentration is determined.

It may be understood that, because a measurement discrepancy or a calculation discrepancy of the tissue fluid glucose concentration is reduced, a discrepancy of a blood glucose concentration determined based on the tissue fluid glucose concentration can be reduced.

Optionally, in some implementations of this application, according to the blood glucose measurement method provided in this application, different blood glucose alert statuses are further classified based on the blood glucose concentration. In different blood glucose alert statuses, manners of alerting the user are different.

It may be understood that, after the blood glucose concentration of the user deviates from a normal value, different alerting manners do not excessively disturb the user, and may also notify, in time, the user or a relative bound to the user of the blood glucose concentration of the user.

Optionally, in some implementations of this application, when it is detected that the ambient temperature changes greatly, or it is detected that the skin temperature changes greatly, or it is detected that the core temperature of the human body changes greatly, the blood glucose alert status is determined based on historical data of the blood glucose concentration. In other words, impact of a temperature may be further considered in a process of determining the blood glucose alert status. The temperature may be the ambient temperature, the skin temperature, and/or the core temperature of the human body.

Optionally, in some implementations of this application, when it is detected that the ambient temperature changes greatly, or it is detected that the skin temperature changes greatly, or it is detected that the core temperature of the human body changes greatly, a blood glucose threshold preset for the blood glucose alert status is dynamically adjusted. For example, an interval of normal blood glucose is narrowed or enlarged, to avoid an increase in a probability of missing detection or a false alarm caused by the temperature. The missing detection means that when the blood glucose concentration of the user is in an interval of an abnormal blood glucose state, because a detected blood glucose concentration is not equal to the blood glucose concentration of the user, the electronic device does not detect that the blood glucose concentration of the user is in the interval of the abnormal blood glucose state. Because it is not detected that the blood glucose concentration of the user is in the interval of the abnormal blood glucose, and a corresponding blood glucose alerting manner does not change, the user cannot be effectively alerted instantly. The false alarm means that when the blood glucose concentration of the user is in the interval of the normal blood glucose state, because the detected blood glucose concentration is not equal to the blood glucose concentration of the user, the electronic device detects that the blood glucose concentration of the user is in the interval of the abnormal blood glucose state. Because it is detected that the blood glucose concentration of the user is in the interval of the abnormal blood glucose, a corresponding blood glucose alerting manner changes.

A case in which it is detected that the ambient temperature changes greatly, the skin temperature changes greatly, or the core temperature of the human body changes greatly may be the following case: A value detected by the electronic device 100 or another electronic device is incorrect, or the ambient temperature, the skin temperature, or the core temperature of the human body changes greatly.

It may be understood that, when it is detected that the temperature changes greatly, different strategies provided in embodiments of this application are used to adjust the blood glucose alert status. There may be a plurality of reasons why it is detected that the temperature changes greatly. For example, a reason why it is detected that the temperature changes greatly may be related to the electronic device 100, the electronic device 200, or the electronic device 300. For another example, a reason why it is detected that the temperature changes greatly may be that the temperature changes greatly. In this case, the body of the user may have a stress reaction, and consequently the blood glucose concentration of the user changes greatly. For another example, when the temperature changes greatly, a temperature measurement discrepancy may increase.

Therefore, in some strategies, reliability of the detected blood glucose concentration needs to be additionally determined, and then the blood glucose alerting manner can be determined. Alternatively, in some strategies, a blood glucose status needs to be determined based on historical data of the blood glucose concentration of the user, and then the blood glucose alerting status is determined. Alternatively, in some strategies, a threshold of the blood glucose concentration needs to be adjusted to reduce the probability of missing detection.

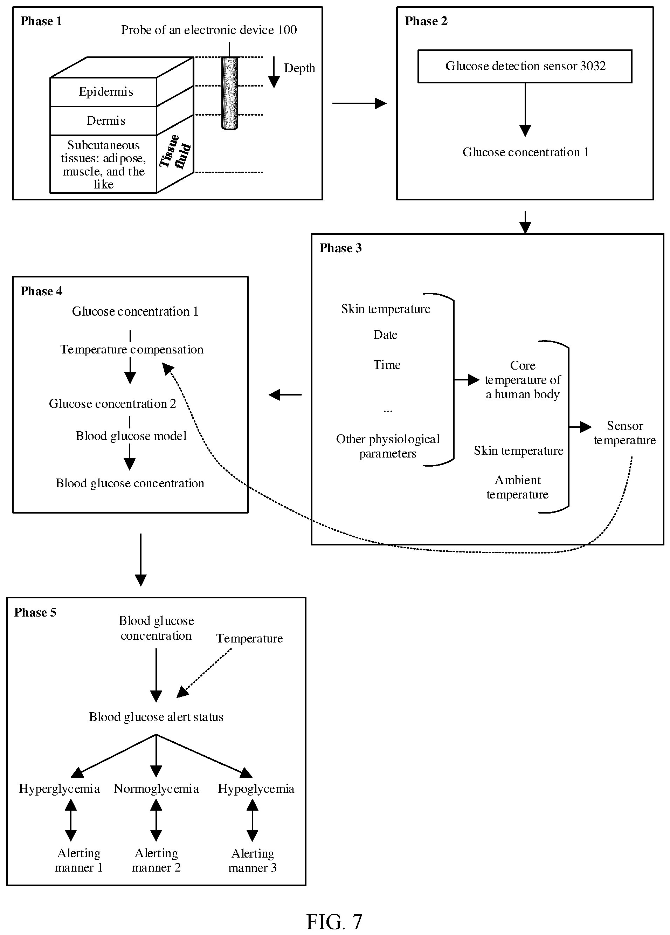

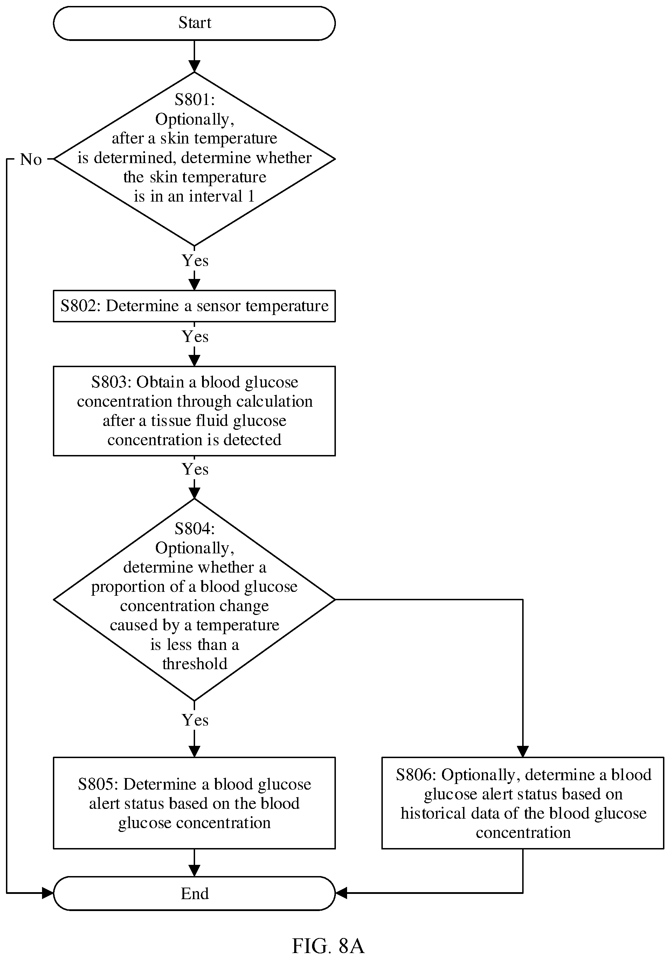

With reference to content shown in FIG. 7 and FIG. 8A, the following describes an example of a blood glucose measurement method provided in embodiments of this application.

FIG. 7 is a diagram of an example of a blood glucose measurement method according to an embodiment of this application.

As shown in FIG. 7, the blood glucose measurement method provided in embodiments of this application may be divided into five phases: Phase 1 to Phase 5.
Phase 1: A user needs to implant an electronic device 100 into a subcutaneous tissue, and then sends a blood glucose electrode into a tissue fluid by using a probe of the electronic device 100, to measure a tissue fluid glucose concentration.
Phase 2: The electronic device 100 determines a glucose concentration 1 by using a glucose detection sensor 3032. For example, a current related to a glucose concentration, for example, a current 1, may be detected in an electrochemical manner by using the glucose detection sensor 3032, and then the glucose concentration 1 is determined based on the current 1.
Phase 3: The electronic device 100, an electronic device 200, and/or an electronic device 300 determine/determines a skin temperature, an ambient temperature, and/or the like. Optionally, in some implementations of this application, the electronic device 200 and/or the electronic device 300 determine/determines parameters such as a date and time. Optionally, in some implementations of this application, the electronic device 200 and/or the electronic device 300 determine/determines a physiological parameter of the user, for example, a gender, an age, or a health status. Some physiological parameters can be obtained only after the user agrees to some privacy agreements.

The electronic device 100, the electronic device 200, or the electronic device 300 determines a core temperature of a human body based on the obtained parameter.

The electronic device 100 or the electronic device 300 determines a sensor temperature based on the core temperature of the human body and the skin temperature; or the electronic device 100 or the electronic device 300 determines a sensor temperature based on the core temperature of the human body, the skin temperature, and the ambient temperature.

An electronic device for determining the core temperature of the human body and an electronic device for determining the sensor temperature may be different electronic devices, or may be a same electronic device.

Phase 4: The electronic device 100 or the electronic device 300 may compensate for the glucose concentration 1 as a glucose concentration 2 based on the sensor temperature. Then, the electronic device 100 or the electronic device 300 determines a blood glucose concentration based on the glucose concentration 2. For example, the blood glucose concentration may be determined by using a blood glucose model.

Optionally, in some implementations of this application, temperature compensation may alternatively be the following: If the electronic device 100 determines a glucose concentration based on a magnitude of a current, the electronic device 100 may first calibrate the current, to directly obtain the glucose concentration 2. For example, the glucose detection sensor 3032 on the electronic device 100 detects the glucose concentration to obtain the current 1, and may obtain a temperature calibration current by using a current-temperature compensation model. The electronic device 100 obtains a current 3 based on the current 1 and a current 2, and then directly obtains the glucose concentration 2 based on the current 3. The current-temperature compensation model may be a model obtained by fitting a linear, polynomial, exponential, or logarithmic function curve or a combination thereof based on temperature-current change data measured in a laboratory.

Optionally, in some implementations of this application, the electronic device 100 or the electronic device 300 may directly determine the blood glucose concentration based on the sensor temperature and the glucose concentration 1.

Phase 5: After the blood glucose concentration is determined, the electronic device 100 or the electronic device 300 may classify different blood glucose alert statuses based on the blood glucose concentration. For example, if the blood glucose concentration is higher than a threshold 1, it is considered that the user has hyperglycemia, and it is determined that an alerting manner is an alerting manner 1; if the blood glucose concentration is lower than a threshold 1 and higher than a threshold 2, it is considered that the user has normoglycemia, and it is determined that an alerting manner is an alerting manner 2; or if the blood glucose concentration is lower than a threshold 2, it is considered that the user has hypoglycemia, and it is determined that an alerting manner is an alerting manner 3.

The alerting manner may be performed by the electronic device 300 or the electronic device 200. On different electronic devices, a same alerting manner may vary. For example, on the electronic device 300, the alerting manner 1 may be a message or a notification that is in a message notification bar and that needs to be viewed. On the electronic device 200, the alerting manner 1 may be vibration.

In the content shown in FIG. 7, a sequence of numbers of the phases does not indicate an execution sequence of the phases. For example, Phase 3 may be performed before Phase 2.

FIG. 8A is a diagram of an example of a blood glucose measurement method according to an embodiment of this application.

S801: Optionally, after a skin temperature is determined, determine whether the skin temperature is in an interval 1.

Any electronic device like an electronic device 100, an electronic device 200, or an electronic device 300 may detect the skin temperature of a user.

After the skin temperature is determined, it is determined whether the skin temperature is in the interval 1. For example, a value range of the interval 1 may be from 20°C to 40°C. Step S802 is performed after it is determined that the skin temperature is in the interval 1.

Optionally, in some implementations of this application, after an ambient temperature is determined, it may be determined whether the ambient temperature is in an interval 2. For example, the interval 2 is from -30°C to 50°C. Step S802 is performed after it is determined that the ambient temperature is in the interval 2.

Optionally, in some implementations of this application, step S802 is performed after it is determined that the skin temperature is in the interval 1 and it is determined that the ambient temperature is in the interval 2.

Optionally, in some implementations of this application, step S802 is performed after it is determined that a change rate of the skin temperature is less than a change rate threshold 1 and/or it is determined that a change rate of the ambient temperature is less than a change rate threshold 2.

Optionally, in some implementations of this application, step S802 is performed after it is determined that the change rate of the skin temperature is less than the change rate threshold 1, it is determined that the change rate of the ambient temperature is less than the change rate threshold 2, it is determined that the skin temperature is in the interval 1, and/or it is determined that the ambient temperature is in the interval 2.

It may be understood that, if the skin temperature is not in the interval 1, a discrepancy of a blood glucose concentration detected in this case may be large. Therefore, blood glucose may not be detected.

S802: Determine a sensor temperature.

In embodiments of this application, the sensor temperature may be determined in a plurality of manners. This is not limited herein.

Optionally, in some implementations of this application, the sensor temperature may be determined based on the skin temperature and a core temperature of a human body. The core temperature of the human body may be accurately estimated based on a skin temperature feature, a skin temperature change time sequence feature, a feature of a difference between the skin temperature and the ambient temperature, and a temperature change rhythm feature by using a machine learning model. A temperature change rhythm means that a temperature change follows a day/night temperature rhythm, a weekly temperature rhythm, and/or an annual temperature rhythm.

Optionally, in some implementations of this application, the sensor temperature may be determined based on the skin temperature, the core temperature of the human body, and the ambient temperature.

Optionally, in some implementations of this application, the sensor temperature may be determined based on the skin temperature and the ambient temperature.

In any one of the foregoing optional implementations, in a process of determining the sensor temperature, time, positioning, and a physiological parameter like a gender, an age, or a health status of the user may be further considered.

In any one of the foregoing optional implementations, in a process of determining the sensor temperature, an implantation position of the electronic device and the like may be further considered.

In any one of the foregoing optional implementations, a method for determining the sensor temperature based on the ambient temperature, the skin temperature, and/or the core temperature may be an interpolation method, a biological heat transfer model, and the like.

The interpolation method may include pre-storing correspondences f1, f2, and the like between the sensor temperature and parameters such as the ambient temperature, the skin temperature, and/or the core temperature, for example, *Tₛₑₙₛₒᵣ = f*1(*Tₑ*, *Tₛ*, *T_{c}*) *= k*1 * *Tₑ + k*2 ** Tₛ + k*3 * *T_{c}.* Herein, *Tₛₑₙₛₒᵣ* is the sensor temperature, *Tₑ* is the ambient temperature, *Tₛ* is the skin temperature, and *T_{c}* is the core temperature of the human body. For another example, *Tₛₑₙₛₒᵣ* = *f*2(*Tₛ*, *T_{c}*) *= k4 * Tₛ + k*5 * *T_{c}.* Herein, *k*1, *k*2, *k*3, *k*4, and *k*5 are coefficients that may be affected by a date, time, positioning, and/or other physiological parameters of the user.

The biological heat transfer model may include the following: A heat dissipation model in which the core temperature of the human body is used as a heat source is first established, and then a thermodynamic equation is established to describe a spatial and temporal temperature distribution of the human body. After an implantation position of the electronic device 100 is determined, the sensor temperature is determined based on the thermodynamic equation and the implantation position of the electronic device 100.

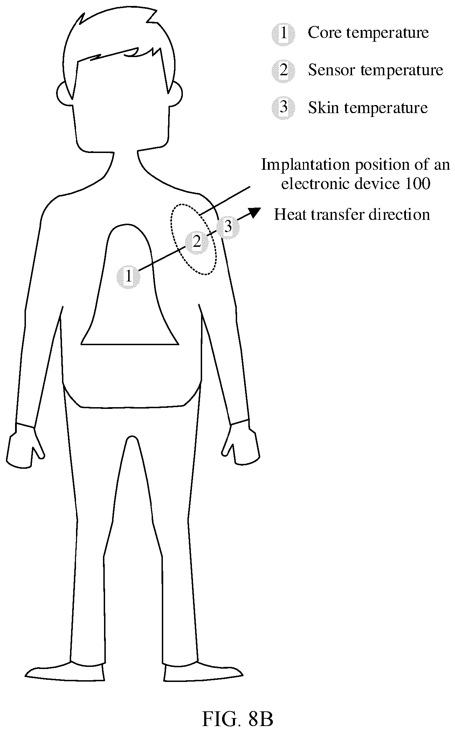

FIG. 8B is a diagram of an example of a biological heat transfer model according to an embodiment of this application.

As shown in FIG. 8B, the implantation position of the electronic device 100 is at an upper left arm of the user. In the biological heat transfer model, the core temperature is used as the heat source, a heat transfer direction is from a chest cavity to the implantation position and then to a skin, and the thermodynamic equation is used to describe a temperature at the implantation position on the human body. The temperature at the implantation position on the human body is the sensor temperature.

Further, if the implantation position may be determined by the electronic device 100 or the electronic device 300, a more accurate sensor temperature may be determined by using the biological heat transfer model.

S803: Obtain a blood glucose concentration through calculation after a tissue fluid glucose concentration is detected.

After the tissue fluid glucose concentration, for example, the glucose concentration 1 in FIG. 7, is detected, the blood glucose concentration is obtained through calculation. The glucose concentration 1 may be compensated for as a glucose concentration 2 based on the sensor temperature, and then the blood glucose concentration is determined based on the glucose concentration 2 and by using a blood glucose model, or the blood glucose concentration may be directly determined based on the sensor temperature and the glucose concentration 1 and by using a blood glucose model.

Optionally, in some implementations of this application, the glucose concentration 2 may be directly obtained, and then the blood glucose concentration is determined, as described in the foregoing content shown in FIG. 7.

S804: Optionally, determine whether a proportion of a blood glucose concentration change caused by a temperature is less than a threshold.

As described in the foregoing content shown in FIG. 1A to FIG. 1D, the temperature affects measurement accuracy of the blood glucose concentration. Therefore, reliability of the detected glucose concentration in step S803 may be determined by determining whether the proportion of the blood glucose concentration change caused by the temperature is less than the threshold. If the proportion of the blood glucose concentration change caused by the temperature is less than the threshold, step S805 is performed; or if the proportion of the blood glucose concentration change caused by the temperature is greater than or equal to the threshold, step S806 is performed.

The proportion of the blood glucose concentration change caused by the temperature may be measured in a plurality of manners. This is not limited herein. In the following, the proportion of the blood glucose concentration change caused by the temperature is referred to as a temperature proportion.

For example, in the content shown in FIG. 7, the electronic device 100 may obtain the temperature calibration current and the current 1, where the current 1 is a current obtained by the glucose detection sensor 3032 by detecting the tissue fluid glucose concentration. In this case, the proportion of the blood glucose concentration change caused by the temperature may be a ratio of the current 1 to the temperature calibration current, or may be a ratio of the current 1 to the current 2, where the current 2 is a sum of the current 1 and the temperature calibration current.

For another example, the proportion of the blood glucose concentration change caused by the temperature may be a ratio of a glucose concentration 3 to the glucose concentration 2, where the glucose concentration 3 is a difference between the glucose concentration 1 and the glucose concentration 2.

For another example, a current change in a past period of time (for example, 30 minutes) is separately calculated as a temperature calibration current change caused by a temperature change and a current 1 change caused by a blood glucose change of the user. In this case, the proportion of the blood glucose concentration change caused by the temperature is a ratio of the temperature calibration current change to the current 1 change, or a ratio of the temperature calibration current to the current 1.

Optionally, in some implementations of this application, before step S804 is performed, it is determined, based on the blood glucose concentration obtained in step S803, whether a blood glucose status changes, for example, changes from a normal blood glucose state to an abnormal blood glucose state, where the abnormal blood glucose state includes a hypoglycemia state, a hyperglycemia state, and the like. Step S804 is performed only when the blood glucose status changes. If the blood glucose status does not change, step S804 may not be performed.

Optionally, in some implementations of this application, step S804 may be performed before step S803. In this case, whether the proportion of the blood glucose concentration change caused by the temperature is less than the threshold is not determined. Instead, whether a proportion of a tissue fluid glucose concentration change caused by the temperature is less than a threshold is determined. If the proportion is less than the threshold, step S803 continues to be performed; or if the proportion is not less than the threshold, the process ends.

It may be understood that, when the blood glucose status does not change, step S804 may not be performed. This is because even if step S804 is performed, regardless of whether the proportion of the blood glucose concentration change caused by the temperature is less than the threshold, after step S805 or step S805 is performed, the blood glucose status and a blood glucose alert status do not change in this case.

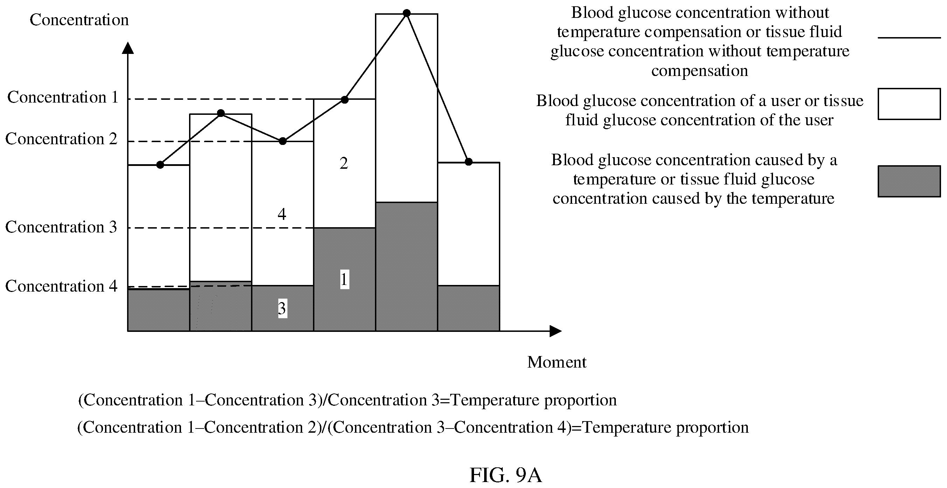

FIG. 9A is a diagram of an example of determining a temperature proportion according to an embodiment of this application.

In the content shown in FIG. 9A, it may be considered that a blood glucose concentration without temperature compensation includes two parts: a blood glucose concentration of a user and a blood glucose concentration caused by the temperature.

For example, in a measurement, a blood glucose concentration without temperature compensation is a concentration 2, and a blood glucose concentration caused by the temperature is a concentration 4. In a next measurement of the measurement, a blood glucose concentration without temperature compensation is a concentration 1, and a blood glucose concentration caused by the temperature is a concentration 4.

In this case, for the second measurement of the two measurements, a temperature proportion may be determined by (concentration 1-concentration 3)/concentration 3, or may be determined by (concentration 1-concentration 2)/(concentration 3-concentration 4). Herein, (concentration 1-concentration 3) is equivalent to an estimation of the blood glucose concentration of the user, concentration 3 is an estimation of the blood glucose concentration caused by the temperature, (concentration 1-concentration 2) is equivalent to an estimation of a blood glucose concentration change of the user, and (concentration 3-concentration 4) is equivalent to an estimation of a blood glucose concentration change caused by a temperature change.

Similarly, in a measurement, a tissue fluid glucose concentration without temperature compensation is a concentration 2, and a tissue fluid glucose concentration caused by the temperature is a concentration 4. In a next measurement of the measurement, a tissue fluid glucose concentration without temperature compensation is a concentration 1, and a tissue fluid glucose concentration caused by the temperature is a concentration 4. Calculation of a temperature proportion is the same as that described above.

The blood glucose concentration caused by the temperature means that because the temperature affects measurement accuracy of the blood glucose concentration, the temperature affects the blood glucose concentration through a discrepancy. In this case, the discrepancy is the blood glucose concentration caused by the temperature, and the discrepancy is also referred to as a delay and/or an amplitude discrepancy in the foregoing description. The temperature does not generate glucose and does not directly affect the blood glucose concentration or the tissue fluid glucose concentration.

S805: Determine the blood glucose alert status based on the blood glucose concentration.

Because reliability of the detected blood glucose concentration is high, the blood glucose alert status may be directly determined based on the blood glucose concentration.

Optionally, in some implementations of this application, based on the foregoing impact of the temperature on accuracy of the blood glucose concentration, blood glucose concentration intervals of different blood glucose alert statuses may be appropriately adjusted.

S806: Optionally, determine the blood glucose alert status based on historical data of the blood glucose concentration.

Because reliability of the detected blood glucose concentration is low, the blood glucose alert status may be determined based on the historical data of the blood glucose concentration.

Optionally, in some implementations of this application, blood glucose concentration intervals of different blood glucose alert statuses may alternatively be adjusted. For example, a blood glucose alert status corresponding to an original blood glucose concentration ranging from A mmol/L to B mmol/L is hyperglycemia, and after it is determined that reliability of the blood glucose concentration is low, a blood glucose concentration interval corresponding to hyperglycemia may be adjusted to A-C mmol/L to B+D mmol/L, or a blood glucose concentration interval corresponding to hyperglycemia may be adjusted to A+E mmol/L to B-F mmol/L. Herein, A, B, C, D, E, and F are positive integers. Whether to enlarge or shrink the blood glucose concentration interval corresponding to hyperglycemia may depend on the physiological parameter like the gender or the health status of the user. This is not limited herein. In this optional implementation, it is not limited to adjust only hyperglycemia, and normoglycemia, hypoglycemia, or the like may also be adjusted.

It may be understood that, step S804 to step S806 are performed, so that when a blood glucose change is mainly caused by the temperature rather than a physiological human body change, a blood glucose concentration interval corresponding to a blood glucose status is changed or the blood glucose alert status is determined based on the historical data of the blood glucose concentration, to reduce incorrect alerts to the user.

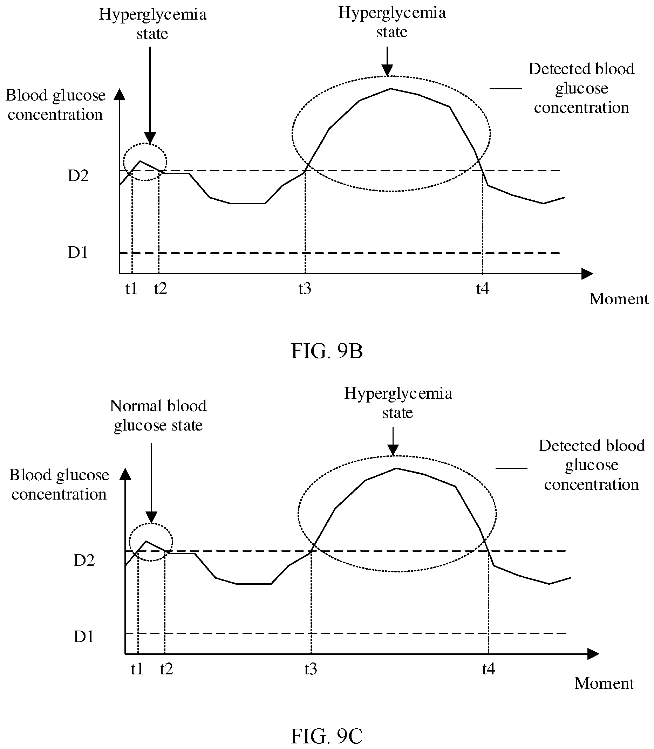

With reference to content shown in FIG. 9B to FIG. 9D, a case in which blood glucose false alarms are reduced in step S804 to step S806 is described as an example.

FIG. 9B to FIG. 9D are diagrams of examples of a blood glucose concentration and a blood glucose status according to an embodiment of this application.

As shown in FIG. 9B and FIG. 9C, at t1 to t2, a blood glucose concentration is higher than a threshold D2; at t3 to t4, the blood glucose concentration is higher than a threshold D2; and at other moments, the blood glucose concentration is lower than the threshold D2 and higher than the threshold D1.

If a blood glucose status is directly determined based on the blood glucose concentration, when the blood glucose concentration is higher than the threshold D2, the blood glucose status is a hyperglycemia state; when the blood glucose concentration is lower than the threshold D2 and higher than the threshold D1, the blood glucose status is a normal blood glucose state; and when the blood glucose concentration is lower than the threshold D1, the blood glucose status is a hypoglycemia state.

As shown in FIG. 9B, the blood glucose status is a hyperglycemia state from t1 to t2.

As shown in FIG. 9C, according to the foregoing step S804 to step 806, at a moment t1, a blood glucose concentration change causes or may cause a blood glucose status change, and it is determined that a proportion of the blood glucose concentration change caused by a temperature is greater than a threshold in this case. In this case, it is determined, based on historical data of the blood glucose concentration, that the blood glucose status is the normal blood glucose state.

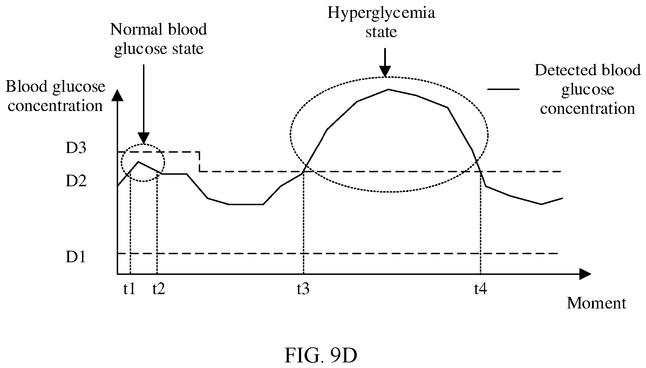

As shown in FIG. 9D, after it is determined that the proportion of the blood glucose concentration change caused by the temperature is greater than the threshold, the blood glucose concentration corresponding to hyperglycemia may be modified. For example, when the proportion of the blood glucose concentration change caused by the temperature is greater than the threshold, a threshold of the hyperglycemia is temporarily modified from D2 to D3, where D3 is higher than D2. The blood glucose status is hyperglycemia only when the blood glucose concentration is higher than D3.

Content shown in FIG. 9C is compared with content shown in FIG. 9D. According to a rule of continuous changes of blood glucose, when the blood glucose concentration change crosses a preset threshold, conditions such as whether the proportion of the blood glucose concentration change caused by the temperature is less than the threshold and whether the blood glucose concentration exceeds a sum of the preset threshold and an offset are determined, to avoid a transition of the blood glucose status and reduce a false alarm probability.

Content shown in FIG. 7 to FIG. 9D describes, as an example, a process of generating some data and functions of the some data in the blood glucose measurement method provided in embodiments of this application. In embodiments of this application, in different cases, a piece of data may be obtained by the electronic device 100 or the electronic device 300, to affect an electronic device that finally determines the blood glucose concentration, and the like. Therefore, the following describes, from perspectives of different electronic devices, examples of several blood glucose measurement methods provided in embodiments of this application.

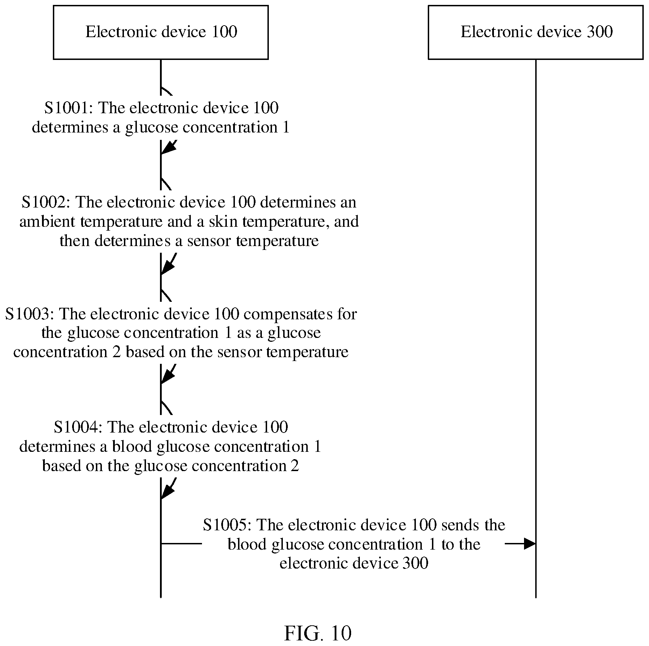

FIG. 10 is a diagram of another example of a blood glucose measurement method according to an embodiment of this application.

S1001: An electronic device 100 determines a glucose concentration 1.

The electronic device 100 detects a tissue fluid glucose concentration by using a glucose detection sensor 3032, to obtain the glucose concentration 1.

Optionally, in some implementations of this application, the electronic device 100 detects a glucose concentration by using the glucose detection sensor 3032 only when the electronic device 100 determines that a skin temperature is in an interval 1. For example, the electronic device 100 detects the glucose concentration only when the electronic device 100 determines that the skin temperature is from 20°C to 40°C. The electronic device 100 may determine the skin temperature by using a temperature electrode 1 or another electronic device like an electronic device 200 or an electronic device 300.

S1002: The electronic device 100 determines an ambient temperature and the skin temperature, and then determines a sensor temperature.

Optionally, in some implementations of this application, step S1002 may be performed before step S1001.

The electronic device 100 may determine the sensor temperature by using a plurality of parameters and in a plurality of manners. This is not limited herein.

Optionally, in some implementations of this application, the electronic device 100 may determine the skin temperature by using the temperature electrode 1, and determine the ambient temperature by using a temperature electrode 2. Then, the electronic device 100 determines a core temperature of a human body based on the skin temperature and the ambient temperature. Then, the electronic device 100 determines the sensor temperature based on the core temperature of the human body and the skin temperature; or the electronic device 100 determines the sensor temperature based on the core temperature of the human body, the skin temperature, and/or the ambient temperature.

Optionally, in some implementations of this application, the electronic device 100 may obtain the ambient temperature and/or the skin temperature from the electronic device 200 or the electronic device 300, and then determine the sensor temperature.

Optionally, in some implementations of this application, the electronic device 100 may obtain the ambient temperature, the skin temperature, and/or the core temperature from the electronic device 200 or the electronic device 300, and then determine the sensor temperature.

Optionally, in some implementations of this application, the electronic device 100 may further obtain a date, time, positioning, and a physiological parameter like a gender or a weight of a user from the electronic device 300, to determine the sensor temperature. The date, the time, and the positioning are used to estimate the core temperature of the user based on a day/night temperature rhythm, a weekly temperature rhythm, and/or an annual temperature rhythm.

In any one of the foregoing optional implementations, a method for determining the sensor temperature based on the ambient temperature, the skin temperature, and/or the core temperature may be an interpolation method, a biological heat transfer model, and the like. For details, refer to the foregoing text descriptions. Details are not described herein again.
S1003: The electronic device 100 compensates for the glucose concentration 1 as a glucose concentration 2 based on the sensor temperature.
Step S1003 is an optional step. If step S1003 is not performed, in step S1004, the electronic device 100 determines a blood glucose concentration 1 based on the glucose concentration 1 and the sensor temperature.

The glucose concentration 2 is considered as the tissue fluid glucose concentration.

S1004: The electronic device 100 determines the blood glucose concentration 1 based on the glucose concentration 2.

The electronic device 100 may determine the blood glucose concentration based on the glucose concentration 2 in a plurality of manners. For details, refer to the foregoing descriptions. Details are not described herein again.

Optionally, in some implementations of this application, the electronic device 100 determines a blood glucose status. In addition, impact of a temperature may be considered in a process of determining the blood glucose status, as described in the foregoing content shown in FIG. 9B to FIG. 9D. Details are not described herein again.

The blood glucose concentration 1 may be referred to as blood glucose data.

S1005: The electronic device 100 sends the blood glucose concentration 1 to the electronic device 300.

Optionally, in some implementations of this application, the electronic device 100 may further determine the blood glucose status and/or a blood glucose alert status based on the blood glucose concentration 1, and then the electronic device 100 sends the blood glucose status and/or the blood glucose alert status to the electronic device 300.

Optionally, in some implementations of this application, the electronic device 100 may further determine the blood glucose status and/or the blood glucose alert status based on the blood glucose concentration 1, and then the electronic device 100 sends the blood glucose status and/or the blood glucose alert status to the electronic device 200, and sends the blood glucose concentration 1 to the electronic device 200.

After receiving the blood glucose concentration 1, the electronic device 300 or the electronic device 200 may determine the blood glucose status and/or the blood glucose alert status based on the blood glucose concentration, and then determine an alerting manner used to alert the user. Alternatively, after receiving the blood glucose status and/or the blood glucose alert status, the electronic device 300 or the electronic device 200 determines an alerting manner used to alert the user. The electronic device 100 further sends the sensor temperature to the electronic device 100 or the electronic device 200.

In a process of determining the blood glucose alert status or the blood glucose status based on the blood glucose concentration 1, the impact of the temperature may be considered, and a result thereof is shown in Table 1.

Table 1 is a diagram of an example of a blood glucose concentration and a blood glucose status according to an embodiment of this application.

**Table 1**

| Blood glucose concentration | Temperature proportion | Blood glucose status |
|---|---|---|
| Concentration 1 | 10% | Determined based on the concentration 1 |
| Concentration 1 | 85% | Determined based on historical data of a blood glucose concentration, or determined after a blood glucose concentration threshold corresponding to the blood glucose status is adjusted |

In the content shown in Table 1, the temperature proportion is a proportion of a blood glucose concentration change caused by a temperature. For details, refer to the text description in step S804. Details are not described herein again.

It may be understood that, in the blood glucose measurement method shown in FIG. 10, after the electronic device 100 determines the blood glucose concentration 1, the electronic device 100 and/or the electronic device 300 determine/determines the blood glucose alert status, and then determine/determines the alerting manner.

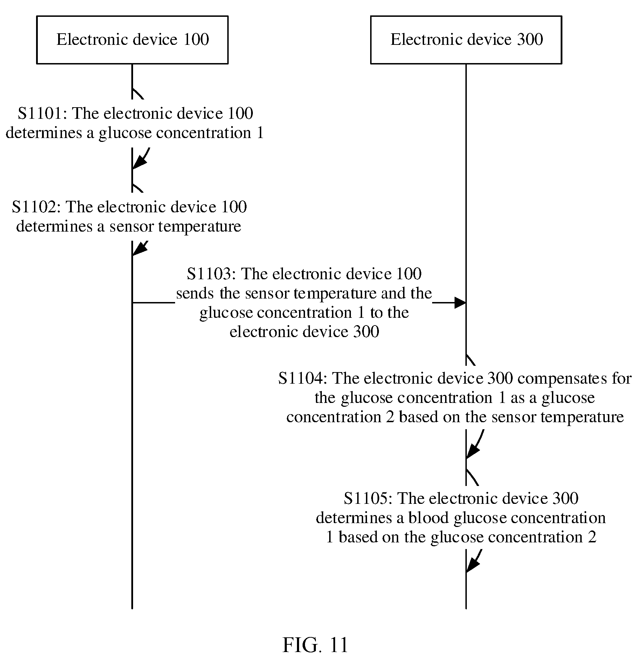

The following uses content shown in FIG. 11 as an example to describe a blood glucose measurement method in which an electronic device 100 determines a parameter like a tissue fluid glucose concentration, and an electronic device 300 determines a blood glucose concentration.

FIG. 11 is a diagram of another example of a method procedure of a blood glucose measurement method according to an embodiment of this application.

S1101: The electronic device 100 determines a glucose concentration 1.

The electronic device 100 determines the glucose concentration 1 by using a glucose detection sensor 3032.

S1102: The electronic device 100 determines a sensor temperature.

For a manner in which the electronic device 100 determines the sensor temperature, refer to the foregoing text descriptions in FIG. 10 and FIG. 7. Details are not described herein again.

S1103: The electronic device 100 sends the sensor temperature and the glucose concentration 1 to the electronic device 300.

The glucose concentration 1 may be referred to as blood glucose data.

S1104: The electronic device 300 compensates for the glucose concentration 1 as a glucose concentration 2 based on the sensor temperature.

Step S1104 is an optional step. When step S1104 is not performed, in step S1105, the electronic device 300 determines a blood glucose concentration based on the glucose concentration 1 and the sensor temperature.

For a manner in which the electronic device 300 compensates for the glucose concentration 1 as the glucose concentration 2 based on the sensor temperature, refer to the foregoing text descriptions in FIG. 10 and FIG. 7. Details are not described herein again.

S1105: The electronic device 300 determines a blood glucose concentration 1 based on the glucose concentration 2.

For a manner in which the electronic device 300 determines the blood glucose concentration based on the glucose concentration 2, refer to the foregoing text descriptions in FIG. 10 and FIG. 7. Details are not described herein again.

After step S1105 is performed, the electronic device 300 may determine a blood glucose status and/or a blood glucose alert status based on the blood glucose concentration 1, or may consider impact of a temperature in a process of determining a blood glucose alert status or a blood glucose status based on the blood glucose concentration 1, as described in step S804 to step S806. Details are not described herein again. A temperature proportion may be determined by the electronic device 100 and sent to the electronic device 300.

Content shown in FIG. 10 and content shown in FIG. 11 are compared, so that it may be understood that, because the electronic device 300 is responsible for a main calculation amount, for example, calculating the glucose concentration 2 and the blood glucose concentration, load of the electronic device 100 can be effectively reduced, and this reduces power consumption of the electronic device 100 and prolongs working time of the electronic device 100. Further, because the electronic device 100 is used as a wearable device or a medical device, a volume of the electronic device 100 determines that a battery capacity of the electronic device 100 is small, and the electronic device 100 may not be charged, it is meaningful to reduce power consumption of the electronic device 100.

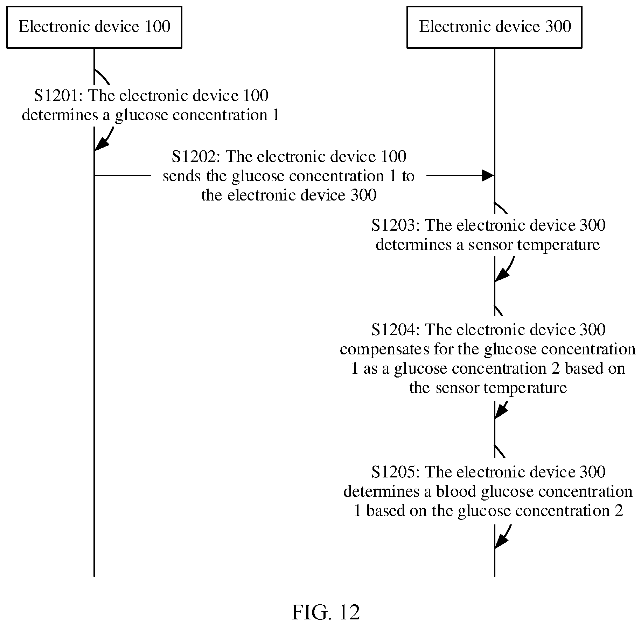

The following uses content shown in FIG. 12 as an example to describe a blood glucose measurement method in which an electronic device 100 determines a tissue fluid glucose concentration, and an electronic device 300 or an electronic device 200 determines a sensor temperature and a blood glucose concentration.

In some implementations, because the electronic device 100 does not have a temperature sensor 3031, or power consumption of the electronic device 100 is considered, the temperature sensor 3031 does not work to reduce the power consumption of the electronic device 100 as much as possible. In this implementation, a temperature like a skin temperature, an ambient temperature, and/or a core temperature is obtained by the electronic device 200 or the electronic device 300.

FIG. 12 is a diagram of another example of a blood glucose measurement method according to an embodiment of this application.

S1201: The electronic device 100 determines a glucose concentration 1.

S1202: The electronic device 100 sends the glucose concentration 1 to the electronic device 300.

Optionally, in some implementations of this application, the electronic device 100 may further determine a skin temperature, an ambient temperature, and/or a core temperature. In addition, the electronic device 100 sends the skin temperature, the ambient temperature, and/or the core temperature to the electronic device 300.

The glucose concentration 1 may be referred to as blood glucose data.

S1203: The electronic device 300 determines a sensor temperature.

The electronic device 300 may determine the skin temperature, the ambient temperature, and/or the core temperature, and further determine the sensor temperature, as shown in the foregoing content. Details are not described herein again. For example, the electronic device 300 may determine the skin temperature, the ambient temperature, and/or the core temperature by using a temperature sensor 180J and an infrared sensor.

Optionally, in some implementations of this application, the electronic device 300 may determine the sensor temperature based on the skin temperature, the ambient temperature, and/or the core temperature sent by the electronic device 100, as shown in the foregoing content. Details are not described herein again.

S1204: The electronic device 300 compensates for the glucose concentration 1 as a glucose concentration 2 based on the sensor temperature.

Step S1204 is an optional step.

When step S1204 is not performed by the electronic device 300, in step S1205, the electronic device 300 determines a blood glucose concentration 1 based on the glucose concentration 1 and the sensor temperature.

S1205: The electronic device 300 determines the blood glucose concentration 1 based on the glucose concentration 2.

For a manner in which the electronic device 300 determines the blood glucose concentration based on the glucose concentration 2, refer to the foregoing text descriptions in FIG. 10 and FIG. 7. Details are not described herein again.

After step S1205 is performed, the electronic device 300 may determine a blood glucose status and/or a blood glucose alert status based on the blood glucose concentration 1, or may consider impact of a temperature in a process of determining a blood glucose alert status or a blood glucose status based on the blood glucose concentration 1, as described in step S804 to step S806. Details are not described herein again. A temperature proportion may be determined by the electronic device 300.

It may be understood that, in the content shown in FIG. 12, the electronic device 300 bears most of the calculation load, for example, determines the blood glucose concentration 1 and the temperature proportion, and the electronic device 100 does not need to calculate the blood glucose concentration 1 or the like. This reduces load of the electronic device 100.

Content shown in FIG. 10 to FIG. 12 above describes an example of a procedure of an interaction between the electronic device 100 and the electronic device 300 in the blood glucose measurement method. The following describes an example of an interaction between the electronic device 100, the electronic device 200, and the electronic device 300 in the blood glucose measurement method.

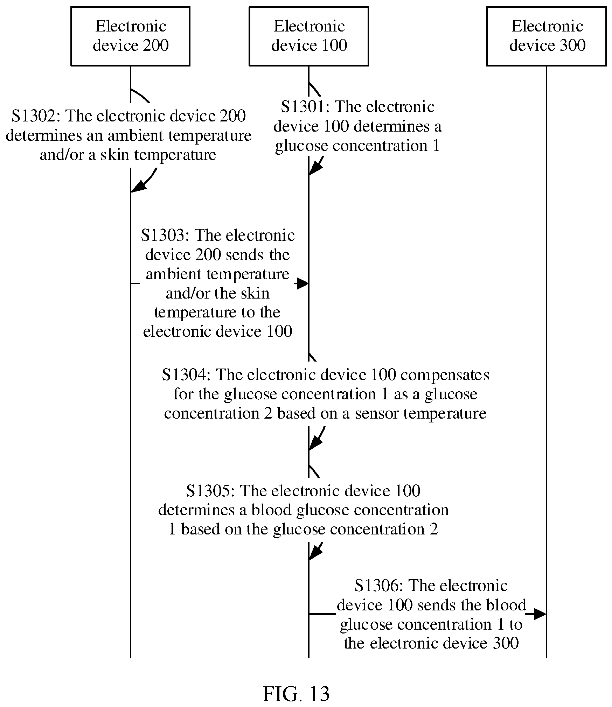

FIG. 13 is a diagram of another example of a blood glucose measurement method according to an embodiment of this application.

S1301: An electronic device 100 determines a glucose concentration 1.

An execution sequence between step S1301 and step S1302 is not limited.

S1302: An electronic device 200 determines an ambient temperature and/or a skin temperature.

The electronic device 200 may determine the ambient temperature and/or the skin temperature by using a temperature sensor 4031.

Optionally, in some implementations of this application, the electronic device 200 may periodically determine the ambient temperature and/or the skin temperature. Optionally, in some implementations of this application, the electronic device 200 determines the ambient temperature and/or the skin temperature in response to receiving a message sent by an electronic device 300 or the electronic device 100.

S1303: The electronic device 200 sends the ambient temperature and/or the skin temperature to the electronic device 100.

S1304: The electronic device 100 compensates for the glucose concentration 1 as a glucose concentration 2 based on a sensor temperature.

For a manner in which the electronic device 100 determines the sensor temperature, refer to the foregoing description. Details are not described herein again.

Step S1304 is an optional step. When step S1304 is not performed, in step S1305, the electronic device 100 determines a blood glucose concentration 1 based on the glucose concentration 1 and the sensor temperature.

S1305: The electronic device 100 determines the blood glucose concentration 1 based on the glucose concentration 2.

The blood glucose concentration 1 may be referred to as blood glucose data.

S1306: The electronic device 100 sends the blood glucose concentration 1 to the electronic device 300.

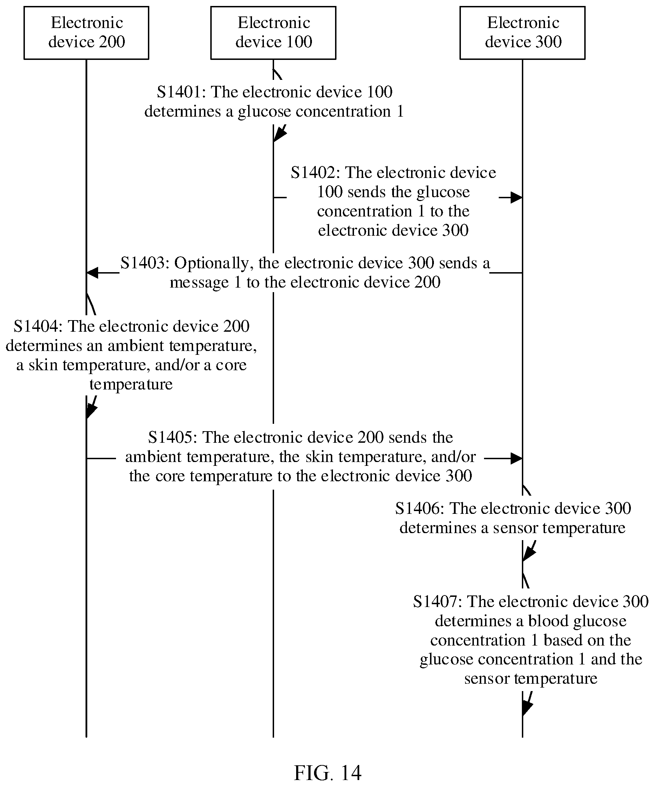

FIG. 14 is a diagram of another example of a blood glucose measurement method according to an embodiment of this application.

S1401: An electronic device 100 determines a glucose concentration 1.

S1402: The electronic device 100 sends the glucose concentration 1 to an electronic device 300.

The glucose concentration 1 may be referred to as blood glucose data.

S1403: Optionally, the electronic device 300 sends a message 1 to an electronic device 200.

Step S1403 is an optional step. The electronic device 300 sends the message 1 to the electronic device 200, to trigger the electronic device 200 to determine an ambient temperature, a skin temperature, and/or a core temperature.

Optionally, in some implementations of this application, the electronic device 100 sends a message 2 to the electronic device 200, to trigger the electronic device 200 to determine the ambient temperature, the skin temperature, and/or the core temperature.

In other words, the electronic device 200 may be triggered by the electronic device 100 or the electronic device 300 to determine the ambient temperature, the skin temperature, and/or the core temperature.

S1404: The electronic device 200 determines the ambient temperature, the skin temperature, and/or the core temperature.

If step S1403 is performed, the electronic device 200 determines the ambient temperature, the skin temperature, and/or the core temperature in response to receiving the message 1. Alternatively, optionally, in some implementations of this application, the electronic device 200 periodically determines the ambient temperature, the skin temperature, and/or the core temperature.

For example, when the electronic device 100 has been connected to the electronic device 300, the electronic device 100 exists in a device list of the electronic device 300 during normal use of the electronic device 100. In this case, the electronic device 300 may send a message 2 to the electronic device 200, so that the electronic device 200 periodically determines the ambient temperature, the skin temperature, and/or the core temperature. This is because the electronic device 100 always periodically or frequently measures the glucose concentration 1, a glucose concentration 2, or a blood glucose concentration 1, and further detects a blood glucose concentration of a user in real time. Therefore, the electronic device 100 may send the message 2 to the electronic device 200, so that the electronic device 200 periodically determines the ambient temperature, the skin temperature, and/or the core temperature. In this way, temperature data does not expire, and an accurate blood glucose concentration is obtained.

S1405: The electronic device 200 sends the ambient temperature, the skin temperature, and/or the core temperature to the electronic device 300.

Optionally, in some implementations of this application, after the electronic device 200 sends the ambient temperature and the skin temperature to the electronic device 300, the electronic device 300 may determine the core temperature based on parameters such as the ambient temperature and the skin temperature.

It may be understood that a more accurate core temperature may be determined if the core temperature is determined by the electronic device 300 instead of the electronic device 200. An estimation discrepancy caused by different core temperatures of users of different age groups, genders, and living habits may be amplified in a process of determining a sensor temperature. Consequently, a more accurate blood glucose temperature cannot be determined. In other words, a part of a discrepancy of the blood glucose concentration comes from a discrepancy of the core temperature. The electronic device 300 may determine the core temperature based on parameters such as a physiological parameter of the user, the ambient temperature, and the skin temperature.

For example, in the foregoing manner of determining the sensor temperature by using the interpolation method, an amplification multiple of the discrepancy is related to a coefficient. However, in the manner of determining the sensor temperature by using the biological heat transfer model, because establishment of the model may be related to a gradient, if an estimated core temperature is near an inflection point of the biological heat transfer model, the discrepancy of the blood glucose concentration is sharply amplified.

S1406: The electronic device 300 determines the sensor temperature.

For a manner in which the electronic device 300 determines the sensor temperature, refer to the foregoing description. Details are not described herein again.

S1407: The electronic device 300 determines the blood glucose concentration 1 based on the glucose concentration 1 and the sensor temperature.

The electronic device 300 determines the blood glucose concentration 1 based on the glucose concentration 1 and the sensor temperature.

Optionally, in some implementations of this application, the electronic device 300 determines the glucose concentration 2 based on the glucose concentration 1 and the sensor temperature, and further determines the blood glucose concentration 1 based on the glucose concentration 2.

It may be understood that, in the content shown in FIG. 13 and FIG. 14, the electronic device 200 determines the ambient temperature, the skin temperature, and/or the core temperature, and the electronic device 100 does not need to determine the ambient temperature, the skin temperature, and/or the core temperature. This helps miniaturize the electronic device 100 and improve a battery life of the electronic device 100.

According to the context, the term "when" used in the foregoing embodiments may be interpreted as a meaning of "if", "after", "in response to determining", or "in response to detecting". Similarly, according to the context, the phrase "when it is determined that..." or "if (a stated condition or event) is detected" may be interpreted as a meaning of "if it is determined that...", "in response to determining...", "when (a stated condition or event) is detected", or "in response to detecting (a stated condition or event)".

All or a part of the foregoing embodiments may be implemented by using software, hardware, firmware, or any combination thereof. When software is used to implement the embodiments, all or a part of the embodiments may be implemented in a form of a computer program product. The computer program product includes one or more computer instructions. When the computer program instructions are loaded and executed on a computer, all or some of the procedures or functions according to embodiments of this application are generated. The computer may be a general-purpose computer, a dedicated computer, a computer network, or another programmable apparatus. The computer instructions may be stored in a computer-readable storage medium or may be transmitted from a computer-readable storage medium to another computer-readable storage medium. For example, the computer instructions may be transmitted from a website, computer, server, or data center to another website, computer, server, or data center in a wired (for example, a coaxial cable, an optical fiber, or a digital subscriber line) or wireless (for example, infrared, radio, or microwave) manner. The computer-readable storage medium may be any usable medium accessible by the computer, or a data storage device, for example, a server or a data center, integrating one or more usable media. The usable medium may be a magnetic medium (for example, a floppy disk, a hard disk, or a magnetic tape), an optical medium (for example, a DVD), a semiconductor medium (for example, a solid-state drive), or the like.

Persons of ordinary skill in the art may understand that all or some of the processes of the methods in embodiments may be implemented by a computer program instructing related hardware. The program may be stored in a computer-readable storage medium. When the program is executed, the processes of the methods in embodiments may be performed. The foregoing storage medium includes any medium that can store program code, for example, a ROM, a random access memory RAM, a magnetic disk, or an optical disc.

## Claims

1. A blood glucose measurement method, applied to a first electronic device, wherein the first electronic device comprises a glucose detection sensor and a temperature detection sensor, the temperature detection sensor is configured to detect a skin temperature, the temperature detection sensor is located outside a body of a user after a detection element of the glucose detection sensor of the first electronic device is implanted into a subcutaneous tissue of the user, the glucose detection sensor is configured to detect a glucose concentration, and the method comprises:
determining, by the first electronic device, a core temperature based on a first parameter after the detection element of the glucose detection sensor of the first electronic device is implanted into the subcutaneous tissue of the user, wherein the first parameter comprises the skin temperature, the first parameter further comprises a second parameter, the second parameter comprises time, a location, and/or a physiological parameter of the user, the second parameter is obtained by the first electronic device from a second electronic device, and the core temperature comprises a temperature of an abdominal cavity or a chest cavity of the user;
determining, by the first electronic device, a sensor temperature based on the core temperature and the skin temperature, wherein the sensor temperature is a temperature of the subcutaneous tissue in which the detection element is located; and
determining, by the first electronic device, a blood glucose concentration of the user based on a reading of the glucose detection sensor and the sensor temperature.

2. The method according to claim 1, wherein the determining, by the first electronic device, a blood glucose concentration of the user based on a reading of the glucose detection sensor and the sensor temperature specifically comprises:
determining, by the first electronic device, a first current intensity based on the glucose detection sensor;
determining, by the first electronic device, a second current intensity based on the sensor temperature;
calibrating, by the first electronic device, the first current intensity to a third current intensity based on the second current intensity; and
determining, by the first electronic device, the blood glucose concentration of the user based on the third current intensity.

3. The method according to claim 1, wherein the determining, by the first electronic device, a blood glucose concentration of the user based on a reading of the glucose detection sensor and the sensor temperature specifically comprises:
determining, by the first electronic device, the glucose concentration based on the glucose detection sensor; and
determining, by the first electronic device, the blood glucose concentration of the user based on the glucose concentration and the sensor temperature.

4. The method according to claim 2 or 3, wherein after the determining, by the first electronic device, a blood glucose concentration of the user based on a reading of the glucose detection sensor and the sensor temperature, the method further comprises:
when a first condition is met, determining, by the first electronic device, a blood glucose status based on the blood glucose concentration of the user, wherein the blood glucose status comprises hyperglycemia and/or hypoglycemia.

5. The method according to claim 4, wherein after the determining, by the first electronic device, a blood glucose concentration of the user based on a reading of the glucose detection sensor and the sensor temperature, the method further comprises:
when the first condition is not met, determining, by the first electronic device, the blood glucose status based on historical data of the blood glucose concentration.

6. The method according to claim 4 or 5, wherein
the first condition is that a first proportion is less than or equal to a threshold, and the first proportion is a proportion of a blood glucose concentration change caused by the sensor temperature.

7. The method according to claim 2 or 3, wherein after the first electronic device determines a first concentration based on the reading of the glucose detection sensor and the sensor temperature, the method further comprises:
when a first condition is not met, determining, by the first electronic device, a third concentration based on the first concentration and a first offset, and then determining a blood glucose status based on the third concentration, wherein the blood glucose status comprises hyperglycemia and/or hypoglycemia; or
when the first condition is met, determining, by the first electronic device, the blood glucose status based on the first concentration, wherein
the first condition is that a first proportion is less than or equal to a threshold, and the first proportion is a proportion of a blood glucose concentration change caused by the sensor temperature.

8. The method according to any one of claims 1 to 7, wherein before the determining, by the first electronic device, a core temperature based on a first parameter after the detection element of the glucose detection sensor of the first electronic device is implanted into the subcutaneous tissue of the user, the method further comprises:
determining, by the first electronic device, that an ambient temperature is within a first interval, and/or determining, by the first electronic device, that a change rate of the ambient temperature is less than a change rate threshold.

9. A blood glucose measurement method, applied to a system comprising a first electronic device, a second electronic device, and a third electronic device, wherein the first electronic device comprises a glucose detection sensor, the third electronic device comprises a temperature detection sensor, and the method comprises:
determining, by the first electronic device, first blood glucose data based on the glucose detection sensor after a detection element of the glucose detection sensor of the first electronic device is implanted into a subcutaneous tissue of a user;
receiving, by the second electronic device, the first blood glucose data sent by the first electronic device;
receiving, by the second electronic device, a skin temperature sent by the third electronic device, wherein the skin temperature is determined by the third electronic device based on the temperature detection sensor;
determining, by the second electronic device, a core temperature based on the skin temperature and a first parameter, wherein the first parameter comprises time, a location, and/or a physiological parameter of the user, and the core temperature is a temperature of an abdominal cavity or a chest cavity of the user;
determining, by the second electronic device, a sensor temperature based on the skin temperature and the core temperature, wherein the sensor temperature is a temperature of the subcutaneous tissue in which the detection element is located; and
determining, by the second electronic device, a blood glucose concentration of the user based on the sensor temperature and the first blood glucose data.

10. The method according to claim 9, wherein after the receiving, by the second electronic device, the first blood glucose data sent by the first electronic device, the method further comprises:
sending, by the second electronic device, a first message to the third electronic device; and
after the third electronic device receives the first message, determining, by the third electronic device, the skin temperature by using the temperature detection sensor.

11. The method according to claim 9 or 10, wherein after the determining, by the second electronic device, a blood glucose concentration of the user based on the sensor temperature and the first blood glucose data, the method further comprises:
when a first condition is met, determining, by the second electronic device, a blood glucose status based on the blood glucose concentration of the user, wherein the blood glucose status comprises hyperglycemia and/or hypoglycemia.

12. The method according to claim 9 or 10, wherein after the determining, by the second electronic device, a blood glucose concentration of the user based on the sensor temperature and the first blood glucose data, the method further comprises:
when the first condition is not met, determining, by the second electronic device, the blood glucose status based on historical data of the blood glucose concentration of the user.

13. The method according to claim 11 or 12, wherein
the first condition is that a first proportion is less than or equal to a threshold, and the first proportion is a proportion of a blood glucose concentration change caused by the sensor temperature.

14. The method according to claim 9 or 10, wherein after the determining, by the second electronic device, a blood glucose concentration of the user based on the sensor temperature and the first blood glucose data, the method further comprises:
when the first condition is not met, determining, by the second electronic device, a first concentration based on the blood glucose concentration of the user and a first offset, and then determining a blood glucose status based on the first concentration, wherein the blood glucose status comprises hyperglycemia and/or hypoglycemia; or
when the first condition is met, determining, by the first electronic device, the blood glucose status based on the blood glucose concentration of the user, wherein
the first condition is that a first proportion is less than or equal to a threshold, and the first proportion is a proportion of a blood glucose concentration change caused by the sensor temperature.

15. The method according to claim 14, wherein
the first proportion is a ratio of a second concentration to the blood glucose concentration of the user, and the second concentration is a difference between the first concentration and the blood glucose concentration of the user.

16. The method according to any one of claims 9 to 15, wherein before the determining, by the second electronic device, a blood glucose concentration of the user based on the sensor temperature and the first blood glucose data, the method further comprises:
determining, by the second electronic device, that an ambient temperature is within a first interval, and/or determining, by the second electronic device, that a change rate of the ambient temperature is less than a change rate threshold.

17. A blood glucose measurement method, applied to a second electronic device, wherein the method comprises:
receiving, by the second electronic device, first blood glucose data sent by a first electronic device;
receiving, by the second electronic device, a skin temperature sent by the first electronic device and/or a third electronic device;
determining, by the second electronic device, a core temperature based on the skin temperature and a first parameter, wherein the first parameter comprises time, a location, and/or a physiological parameter of a user, and the core temperature is a temperature of an abdominal cavity or a chest cavity of the user;
determining, by the second electronic device, a sensor temperature based on the skin temperature and the core temperature, wherein the sensor temperature is a temperature of a subcutaneous tissue in which the detection element is located; and
determining, by the second electronic device, a blood glucose concentration of the user based on the sensor temperature and the first blood glucose data.

18. The method according to claim 17, wherein after the receiving, by the second electronic device, the first blood glucose data sent by the first electronic device, the method further comprises:
sending, by the second electronic device, a first message to the third electronic device, wherein the first message is used to trigger the third electronic device to determine the skin temperature.

19. The method according to claim 17 or 18, wherein after the determining, by the second electronic device, a blood glucose concentration of the user based on the sensor temperature and the first blood glucose data, the method further comprises:
when a first condition is met, determining, by the second electronic device, a blood glucose status based on the blood glucose concentration of the user, wherein the blood glucose status comprises hyperglycemia and/or hypoglycemia.

20. The method according to claim 17 or 18, wherein after the determining, by the second electronic device, a blood glucose concentration of the user based on the sensor temperature and the first blood glucose data, the method further comprises:
when the first condition is not met, determining, by the second electronic device, the blood glucose status based on historical data of the blood glucose concentration of the user, wherein the blood glucose status comprises hyperglycemia and/or hypoglycemia.

21. The method according to claim 19 or 20, wherein
the first condition is that a first proportion is less than or equal to a threshold, and the first proportion is a proportion of a blood glucose concentration change caused by the sensor temperature.

22. The method according to claim 17 or 18, wherein after the determining, by the second electronic device, a blood glucose concentration of the user based on the sensor temperature and the first blood glucose data, the method further comprises:
when the first condition is not met, determining, by the second electronic device, a first concentration based on the blood glucose concentration of the user and a first offset, and then determining a blood glucose status based on the first concentration, wherein the blood glucose status comprises hyperglycemia and/or hypoglycemia; or
when the first condition is met, determining, by the first electronic device, the blood glucose status based on the blood glucose concentration of the user, wherein
the first condition is that a first proportion is less than or equal to a threshold, and the first proportion is a proportion of a blood glucose concentration change caused by the sensor temperature.

23. The method according to claim 22, wherein
the first proportion is a ratio of a second concentration to the blood glucose concentration of the user, and the second concentration is a difference between the first concentration and the blood glucose concentration of the user.

24. The method according to any one of claims 17 to 23, wherein before the determining, by the second electronic device, a blood glucose concentration of the user based on the sensor temperature and the first blood glucose data, the method further comprises:
determining, by the second electronic device, that an ambient temperature is within a first interval, and/or determining, by the second electronic device, that a change rate of the ambient temperature is less than a change rate threshold.

25. An electronic device, wherein the electronic device comprises one or more processors and a memory; and
the memory is coupled to the one or more processors, the memory is configured to store computer program code, the computer program code comprises computer instructions, and the one or more processors invoke the computer instructions, so that the electronic device performs the method according to any one of claims 17 to 24.

26. An electronic device, wherein the electronic device comprises one or more processors, a memory, a glucose detection sensor, and a temperature sensor, the temperature detection sensor is configured to detect a skin temperature, the temperature detection sensor is located outside a body of a user after a detection element of the glucose detection sensor of the electronic device is implanted into a subcutaneous tissue of the user, and the glucose detection sensor is configured to detect a glucose concentration; and
the memory is coupled to the one or more processors, the memory is configured to store computer program code, the computer program code comprises computer instructions, and the one or more processors invoke the computer instructions, so that the electronic device performs the method according to any one of claims 1 to 8.

27. A chip system, wherein the chip system is used in an electronic device, the chip system comprises one or more processors, and the processor is configured to invoke computer instructions, so that the electronic device performs the method according to any one of claims 1 to 8 or claims 17 to 24.

28. A computer-readable storage medium, comprising instructions, wherein when the instructions are run on an electronic device, the electronic device is enabled to perform the method according to any one of claims 1 to 8 or claims 17 to 24.
